# EUROPÄISCHE PATENTSCHRIFT

(11) **EP 3 873 423 B1**
(45) Veröffentlichungstag und Bekanntmachung des Hinweises auf die Patenterteilung: **04.12.2024**
(21) Anmeldenummer: 19797678.0
(22) Anmeldetag: 31.10.2019
(51) Int. Cl.: A61Q 5/12, A61Q 5/00, A61K 8/58, A61K 8/27, A61K 8/28, A61K 8/19

(54) **BIS(TRIETHOXYSILYLPROPYL)AMIN IN KOMBINATION MIT LANTHANCHLORID**
BIS(TRIETHOXYSILYLPROPYL)AMINE IN COMBINATION WITH LANTHANIUM-CHLORIDE
BIS(TRIÉTHOXYSILYLPROPYL)AMINE EN COMBINAISON AVEC CHLORURE DU LANTHAN

(30) Priorität: 31.10.2018 DE 102018127296
(43) Veröffentlichungstag der Anmeldung: 08.09.2021
(73) Patentinhaber: Henkel AG & Co. KGaA, 40589 Düsseldorf (DE)
(72) Erfinder: KROHN, Rene, 22848 Norderstedt (DE); SCHULZE ZUR WIESCHE, Erik, 33619 Bielefeld (DE)
(86) Internationale Anmeldenummer: PCT/EP2019/079777
(87) Internationale Veröffentlichungsnummer: WO 2020/089363

(56) Entgegenhaltungen:
- DE-A1- 102004 013 795
- US-A1- 2010 083 446
- DATABASE GNPD [online] MINTEL; 21 September 2017 (2017-09-21), ANONYMOUS: "Sweet Moisture Foundation Refill", XP055650922, retrieved from www.gnpd.com Database accession no. 5119969
- DATABASE GNPD [online] MINTEL; 27 October 2017 (2017-10-27), ANONYMOUS: "Strengthening Shampoo", XP055650914, retrieved from www.gnpd.com Database accession no. 5197189
- DATABASE GNPD [online] MINTEL; 20 November 2017 (2017-11-20), ANONYMOUS: "Texturizing Conditioner", XP055650919, retrieved from www.gnpd.com Database accession no. 5255071

## Beschreibung

Die vorliegende Erfindung betrifft ein kosmetisches Mittel zur Behandlung eines keratinischen Materials, wobei das kosmetische Mittel ein Fluid darstellt und mindestens eine organische Siliciumverbindung, insbesondere Bis(triethoxysilylpropyl)amin, und mindestens ein mehrwertiges Metallkation umfasst, sowie die Verwendung des kosmetischen Mittels.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine besondere Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung: Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten.

Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen. Auch häufiges Waschen oder andere Pflegebehandlungen können zu Farbverschiebungen bewirken. Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Bei einem weiteren gebräuchlichen Färbeverfahren werden Vorstufen des natürlichen Haarfarbstoffes Melanin auf das Haar aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss.

Bei allen Haarfärbung treten mehrere Probleme auf. Zum einen kann eine unerwünschte Anfärbung von Hautpartien beim Ausspülen des Haarfärbemittels auftreten. Dieser Effekt kann auch dazu führen, dass hellere "Strähnchen" durch die Haarfarbe beim Auswaschen nachgefärbt werden, oder das Färbemittel beim Auswaschen aus dunkleren "Strähnchen" das hellere Basishaar anfärbt. Das Auswaschen von Farbstoffen ist generell mit dem Nachteil verbunden, dass die ursprünglich gewünschte Farbe sich verändert. Besonders Haarfarben mit Rot- oder Blautönen besitzen eine verkürzte Verweilzeit im Haar, so dass deren Auswaschung zu unerwünschten Farben führen. Diesem Problem wird auch durch Farbschutzprodukte entgegengewirkt.

Zum anderen bedeuten alle chemischen Beanspruchungen eine Erschwerung von Färbeprozessen. Die äußere Beanspruchung der Haare durch chemische Stoffe aus einer Vielzahl unterschiedlicher Quellen stellt die Entwicklung kosmetischer Färbeprodukte vor Herausforderungen. Luft- und Wasserverunreinigungen wirken sich nachteilig auf Haut und Haare aus. Zu den wichtigsten Luftschadstoffen gehören polycyclische aromatische Kohlenwasserstoffe, flüchtige organische Verbindungen, Stickoxide (NOx), Partikel und Zigarettenrauch. Die Wirkung verschiedener Luftschadstoffe kann in Gegenwart anderer Luftschadstoffe und unter Einwirkung von UV-Strahlung verstärkt werden.

Im Stand der Technik sind kosmetische Produkte bekannt, die eine Silan-Verbindung in Kombination mit unterschiedlichen mehrwertigen Metallkationen wie Mg, Zn, Al, Bi, Si, Ti, Fe, Ca, usw. umfassen.

Aus der US 2010/083446 A1 ist ein Haarbehandlungsmittel bekannt, welches ein Silan, ein hydrophobes filmbildendes Polymer, Pigmente und flüchtiges Lösungsmittel enthält. Die DE 10 2004 013795 A1 beschreibt Mittel, die substituierte Aminopolyorganosiloxane enthalten.

Es besteht daher ein Bedarf an Produkten, die eine besondere Pflegewirkung für Haare herbeiführen, um optimale Färbeprozesse zu ermöglichen, und die gewährleisten, dass die Haare gleichzeitig eine lang andauernde, gewünschte Färbung beibehalten.

Die der vorliegenden Erfindung zugrunde liegende Aufgabe besteht in der Bereitstellung eines Produkts mit einer verbesserten Pflege- und/oder Schutzwirkung. Insbesondere lag der vorliegenden Erfindung die Aufgabe zugrunde, ein kosmetisches Mittel bereitzustellen, das strapazierten Haaren eine besondere Pflege zukommen lässt und das den Effekt des Auswaschens oder der Farbveränderung reduziert.

Diese Aufgabe wird gelöst durch ein kosmetisches Mittel zur Behandlung eines keratinischen Materials, wobei das kosmetische Mittel ein Fluid darstellt und
a) mindestens eine organische Siliciumverbindung, wobei die mindestens eine organische Siliciumverbindung eine Verbindung der Formel (I) und mindestens eine organische Siliciumverbindung der Formel (IV) enthält,
   wobei in der organischen Siliciumverbindung der Formel (I)

      R₁R₂N-L-Si(OR₃)ₐ(R₄)_{b} (I),

      - R₁, R₂ beide für ein Wasserstoffatom stehen,
      - L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
      - R₃, R₄ unabhängig voneinander für eine Methylgruppe oder für eine Ethylgruppe stehen,
      - a für die Zahl 3 steht und
      - b für die Zahl 0 steht, und
   wobei in der organischen Siliciumverbindung der Formel (IV)

      R₉Si(OR₁₀)ₖ(R₁₁)ₘ (IV),

      - R₉ für eine C₁-C₁₂-Alkylgruppe steht,
      - R₁₀ für ein Wasserstoffatom oder eine C₁-C₆-Alkylgruppe steht,
      - R₁₁ für eine C₁-C₆-Alkylgruppe steht
      - k für eine ganze Zahl von 1 bis 3 steht, und
      - m für die ganze Zahl 3 - k steht, und
b) mindestens ein mehrwertiges Metallkation, umfassend Lanthan in Form von Lanthanchlorid, in einer Stoffmengenkonzentration von 0,001 mol/L bis 2 mol/L, bevorzugt 0,005 mol/L bis 1,5 mol/L, bevorzugter 0,01 bis 1 mol/L, am meisten bevorzugt 0,02 bis 0,5 mol/L, umfasst.

Unter einem keratinischen Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter einem keratinischen Material das menschliche Haar, die menschliche Haut und menschliche Nägel, insbesondere Finger- und Fußnägel, verstanden. Ganz besonders bevorzugt wird unter keratinischem Material das menschliche Haar, insbesondere Kopf- und/oder Barthaare, verstanden.

Als ersten erfindungswesentlichen Bestandteil enthält das kosmetische Mittel zur Behandlung eines keratinischen Materials mindestens eine organische Siliciumverbindung ausgewählt aus Silanen mit einem, zwei oder drei Siliciumatomen, wobei die organische Siliciumverbindung eine oder mehrere Hydroxylgruppen und/oder hydrolysierbare Gruppen pro Molekül umfasst.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die organische Siliciumverbindungen sind Verbindungen, die ein bis drei Siliciumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die WasserstoffAtome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das Mittel zur Behandlung eines keratinischen Materials enthält mindestens eine organischen Siliciumverbindung, die aus Silanen mit einem, zwei oder drei Siliciumatomen ausgewählt ist, wobei die organische Siliciumverbindung eine oder mehrere Hydroxylgruppen oder hydrolysierbare Gruppen pro Molekül umfasst.

Das Mittel zur Behandlung eines keratinischen Materials weist mindestens eine organische Siliciumverbindung auf, die aus Silanen mit einem, zwei oder drei Siliciumatomen ausgewählt ist, wobei die organische Siliciumverbindung außerdem eine oder mehrere basische Gruppe und eine oder mehrere Hydroxylgruppen oder hydrolysierbare Gruppen pro Molekül umfasst.

Bei dieser basischen Gruppe handelt es sich um eine Aminogruppe, die über einen Linker mit einem Siliciumatom verbunden ist.

Bei der oder den hydrolysierbaren Gruppen handelt es sich bevorzugt um eine Ethoxygruppe oder um eine Methoxygruppe.

Das Mittel zur Behandlung eines keratinischen Materials enthält mindestens eine organische Siliciumverbindung der Formel (I)

R₁R₂N-L-Si(OR₃)ₐ(R₄)_{b} (I),

wobei
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht,

In den organischen Siliciumverbindungen der Formel (I)

R₁R₂N-L-Si(OR₃)ₐ(R₄)_{b} (I),

stehen die Reste R₁ und R₂ beide für ein Wasserstoffatom.

Im Mittelteil der organischen Siliciumverbindung befindet sich die Struktureinheit oder der Linker -L- der für eine lineare oder verzweigte, zweibindige C₁-C₂₀-Alkylengruppe steht.

-L- steht für eine lineare zweibindige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die organischen Siliciumverbindungen der Formel (I)

R₁R₂N-L-Si(OR₃)ₐ(R₄)_{b} (I),

tragen jeweils an einem Ende die Silicium-haltige Gruppierung -Si(OR₃)ₐ(R₄)_{b}.

In der endständigen Struktureinheit -Si(OR₃)ₐ(R₄)_{b} stehen R₃ und R₄ unabhängig voneinander für eine Methylgruppe oder eine Ethylgruppe.

Hierbei steht a für eine ganze Zahl von 3 und b ist b gleich 0.

Der beste Schutz vor den negativen Auswirkungen von Wasser- und/oder Luftverschmutzungen ("Anti-Pollution"-Wirkung) und die beste Pflege von beanspruchtem Haar konnte erhalten werden, wenn das Mittel zur Behandlung eines keratinischen Materials mindestens eine organische Siliciumverbindung der Formel (I) enthält, in welcher die Reste R₃, R₄ unabhängig voneinander für eine Methylgruppe oder für eine Ethylgruppe stehen.

Besonders gut geeignete organische Siliciumverbindungen der Formel (I) sind
- (3-Aminopropyl)triethoxysilan 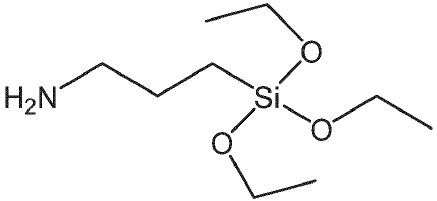
- (3-Aminopropyl)trimethoxysilan 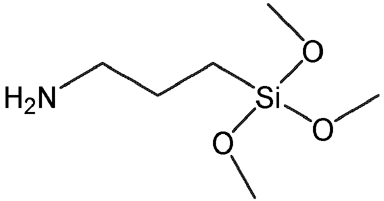
- (2-Aminoethyl)triethoxysilan 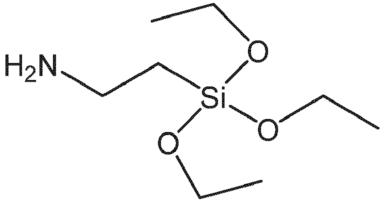
- (2-Aminoethyl)trimethoxysilan 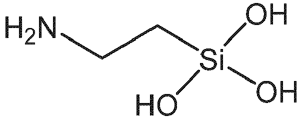
- (3-Dimethylaminopropyl)triethoxysilan 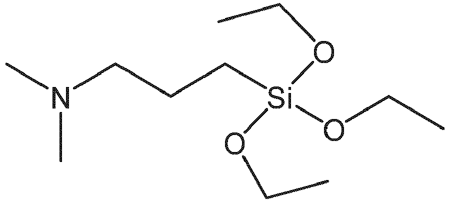
- (3-Dimethylaminopropyl)trimethoxysilan 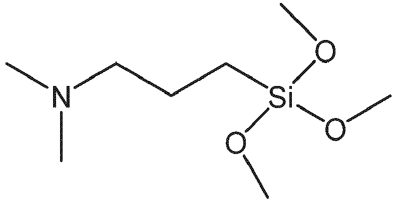
- (2-Dimethylaminoethyl)triethoxysilan und/oder 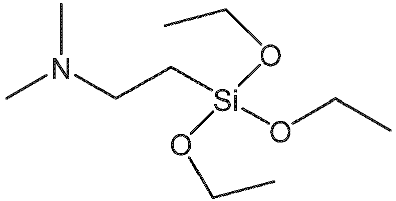
- (2-Dimethylaminoethyl)trimethoxysilan.

Die vorgenannten organische Siliciumverbindung der Formel (I) sind kommerziell erhältlich.

(3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erhältlich.

Im Rahmen einer weiteren Ausführungsform kann das Mittel zur Behandlung eines keratinischen Materials ferner mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II).

Die siliciumorganischen Verbindungen der Formel (II) tragen jeweils an ihren beiden Enden die Siliciumhaltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}-und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Eine sehr hohe Anti-Pollution-Wirkung des Mittels zur Behandlung eines keratinischen Materials konnte erhalten werden, wenn die Reste c und c' beide für die Zahl 3 stehen. In diesem Fall stehen d und d` beide für die Zahl 0.

In einer weiteren bevorzugten enthält das Mittel zur Behandlung eines keratinischen Materials ferner mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅^{,})_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die organischen Siliciumverbindung der Formel (IIa)

(R₅O)₃Si-(A)ₑ-[NR₇-(A')_{f}[O-(A")]_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der "Anti-Pollution"-Wirkung erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die organischen Siliciumverbindung der Formel (Ilb)

(R₅O)_{c}(R₆)_{d}Si-(A)-[NR₇-(A')]-Si(R₆')_{d'}(OR₅')_{c'} (IIb).

Die Reste A, A', A", A‴ und Aʺʺ stehen unabhängig voneinander für eine lineare oder verzweigte, zweibindige C₁-C₂₀-Alkylengruppe. Bevorzugt stehen die Reste A, A', A", A‴ und Aʺʺ unabhängig voneinander für eine lineare, zweibindige C₁-C₂₀-Alkylengruppe. Weiter bevorzugt stehen die Reste A, A', A", A‴ und Aʺʺ unabhängig voneinander für eine lineare zweibindige C₁-C₆-Alkylengruppe. Besonders bevorzugt stehen die Reste A, A', A", A‴ und Aʺʺ unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und Aʺʺ für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III)

- (Aʺʺ)-Si(R₆")_{d}"(OR₅")_{c}" (III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)]. Steht nun der Rest R7 für eine Gruppierung der Formel (III), so enthält das Mittel zur Behandlung eines keratinischen Materials eine organische Siliciumverbindung mit 3 reaktiven Silan-Gruppen.

In einer weiteren bevorzugten enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d}(OR₅')_{c'} (II),

wobei
- e und f beide für die Zahl 1 stehen,
- g und h beide für die Zahl 0 stehen,
- A und A` unabhängig voneinander für eine lineare, zweibindige C₁-C₆-Alkylengruppe stehen
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform enthält das Mittel zur Behandlung eines keratinischen Materials mindestens eine organische Siliciumverbindung der Formel (II), wobei
- e und f beide für die Zahl 1 stehen,
- g und h beide für die Zahl 0 stehen,
- A und A` unabhängig voneinander für eine Methylengruppe (-CH₂-), eine Ethylengruppe (-CH₂-CH₂-) oder eine Propylengruppe (-CH₂-CH₂-CH₂) stehen,
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der Aufgabenstellung gut geeignete organische Siliciumverbindungen der Formel (II) sind
- 3-(Trimethoxysilyl)-N-[3-(trimethoxysilyl)propyl]-1-propanamin 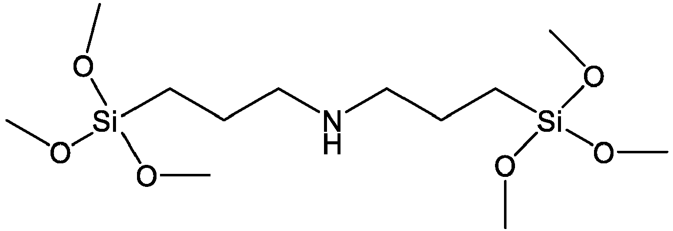
- 3-(Triethoxysilyl)-N-[3-(triethoxysilyl)propyl]-1-propanamin 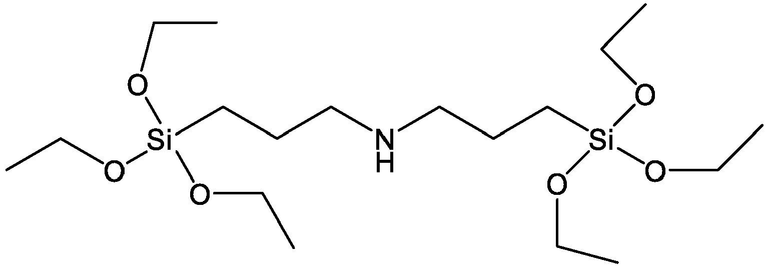
- N-Methyl-3-(trimethoxysilyl)-N-[3-(trimethoxysilyl)propyl]-1-propanamin 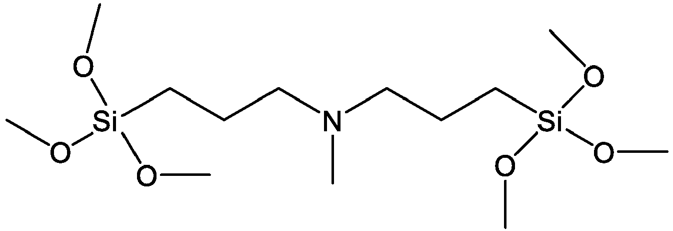
- N-Methyl-3-(triethoxysilyl)-N-[3-(triethoxysilyl)propyl]-1-propanamin 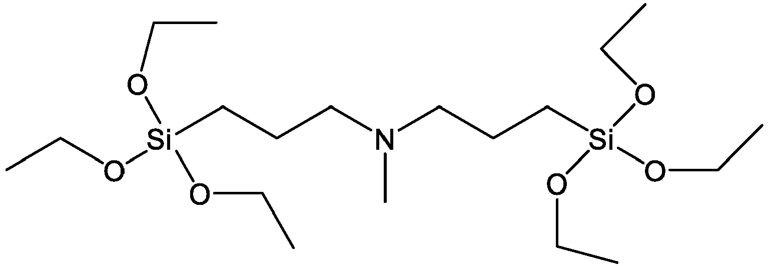
- 2-[Bis[3-(trimethoxysilyl)propyl]amino]-ethanol 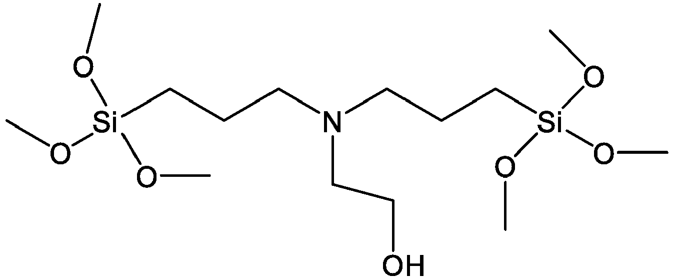
- 2-[Bis[3-(triethoxysilyl)propyl]amino]-ethanol 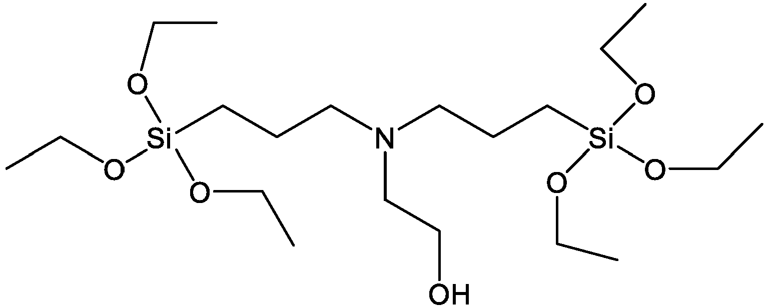
- 3-(Trimethoxysilyl)-N,N-bis[3-(trimethoxysilyl)propyl]-1-propanamin 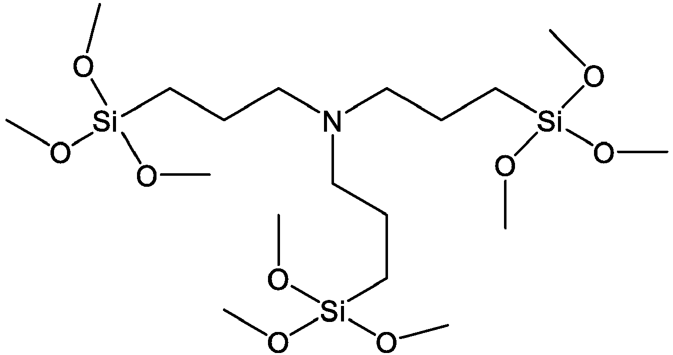
- 3-(Triethoxysilyl)-N,N-bis[3-(triethoxysilyl)propyl]-1-propanamin 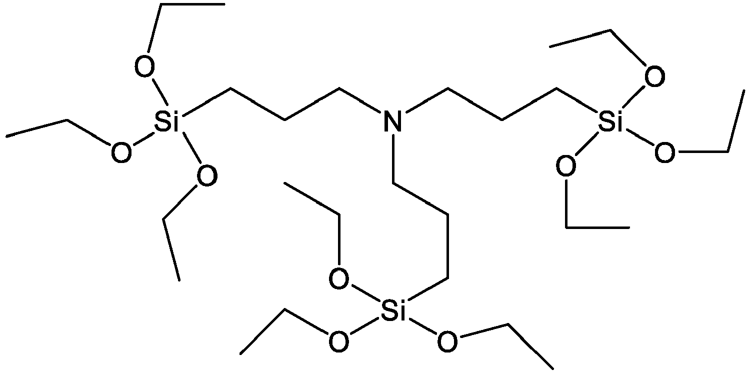
- N 1,N1-Bis[3-(trimethoxysilyl)propyl]-1,2-ethanediamin, 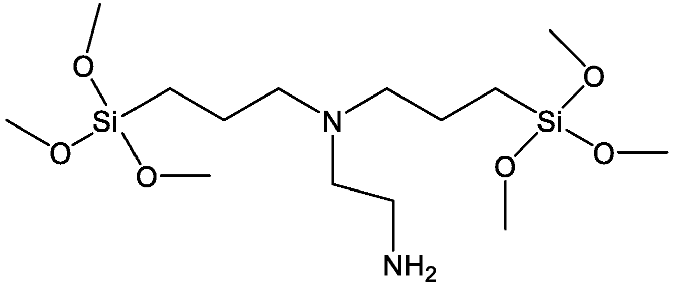
- N1,N1-Bis[3-(triethoxysilyl)propyl]-1,2-ethanediamin, 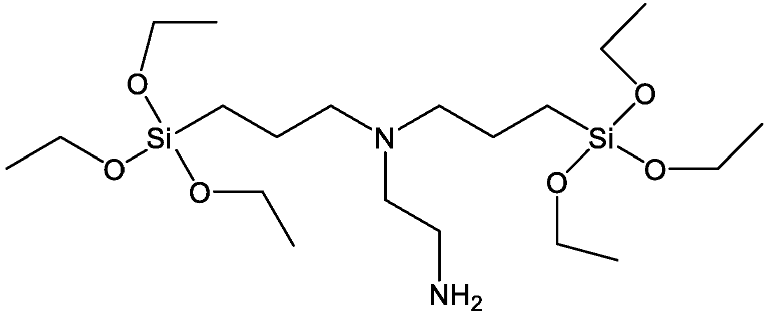
- N,N-Bis[3-(trimethoxysilyl)propyl]-2-propen-1-amin 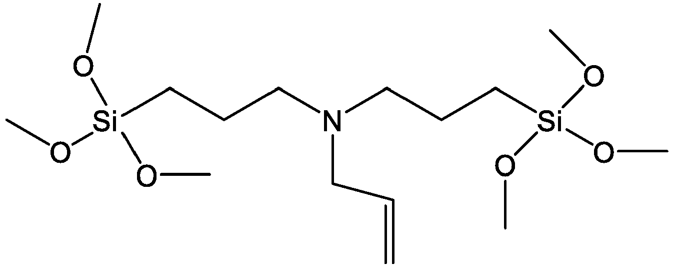
- N,N-Bis[3-(triethoxysilyl)propyl]-2-propen-1-amin

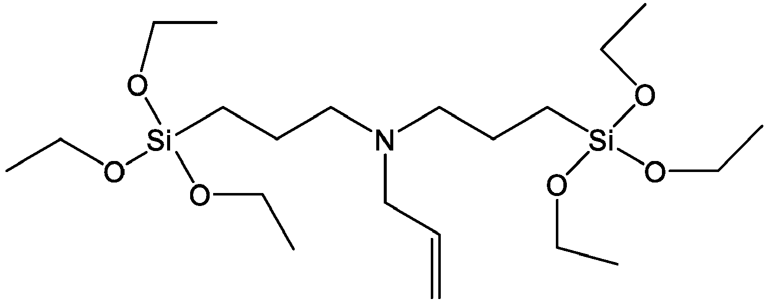

Die vorgenannten organische Siliciumverbindung der Formel (II) sind kommerziell erhältlich.

Bis(trimethoxysilylpropyl)amin mit der CAS-Nummer 82985-35-1 kann beispielsweise von Sigma-Aldrich käuflich erworben werden.

Bis[3-(triethoxysilyl)propyl]amin, auch bezeichnet als 3-(Triethoxysilyl)-N-[3-(triethoxysilyl)propyl]-1-propanamin, mit der CAS-Nummer 13497-18-2 kann zum Beispiel von Sigma-Aldrich käuflich erworben werden oder ist im Handel unter der Produktbezeichnung Dynasylan 1122 von Evonik erhältlich. N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

Das auf dem Haar angewendete Mittel zur Behandlung eines keratinischen Materials enthält mindestens eine organische Siliciumverbindung der Formel (IV)

R₉Si(OR₁₀)ₖ(R₁₁)ₘ (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkylalkoxysilane oder der Alkylhydroxysilane bezeichnet werden,

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform enthält das Mittel zur Behandlung eines keratinischen Materials zusätzlich zu der oder den organischen Siliciumverbindungen der Formel (I) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform enthält das Mittel zur Behandlung eines keratinischen Materials zusätzlich zu den organischen Siliciumverbindungen der Formel (I) und (II) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₂-Alkylgruppe. Diese C₁-C₁₂-Alkylgruppe ist gesättigt und kann linear oder verzweigt sein. Bevorzugt steht R9 für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgruppe oder eine n-Octylgruppe.

In den organischen Siliciumverbindungen der Formel (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen der Formel (IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Sehr vorteilhafte Wirkungen konnten erhalten werden, wenn das Mittel zur Behandlung eines keratinischen Materials mindestens eine organische Siliciumverbindung der Formel (IV) enthält, in welcher der Rest k für die Zahl 3 steht. In diesem Fall steht der Rest m für die Zahl 0.

Zur Lösung der Aufgabenstellung besonders gut geeignete organische Siliciumverbindungen der Formel (IV) sind
- Methyltrimethoxysilan 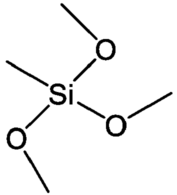
- Methyltriethoxysilan 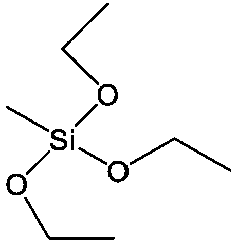
- Ethyltrimethoxysilan 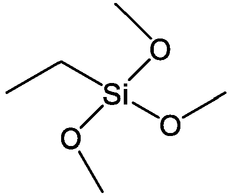
- Ethyltriethoxysilan 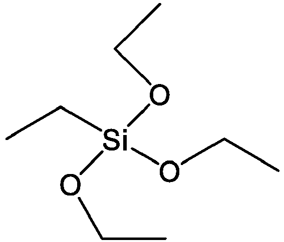
- n-Hexyltrimethoxysilan 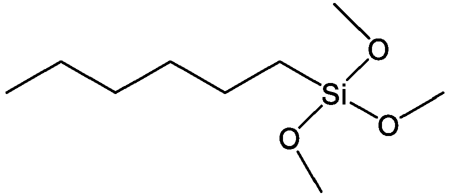
- n-Hexyltriethoxysilan 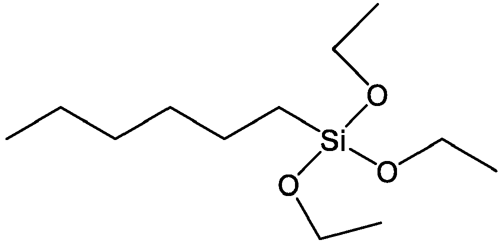
- n-Octyltrimethoxysilan 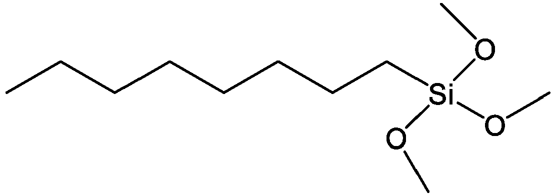
- n-Octyltriethoxysilan 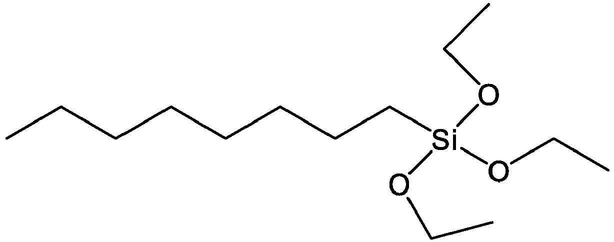
- n-Dodecyltrimethoxysilan und/oder 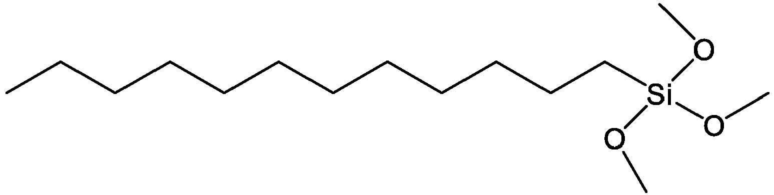
- n-Dodecyltriethoxysilan 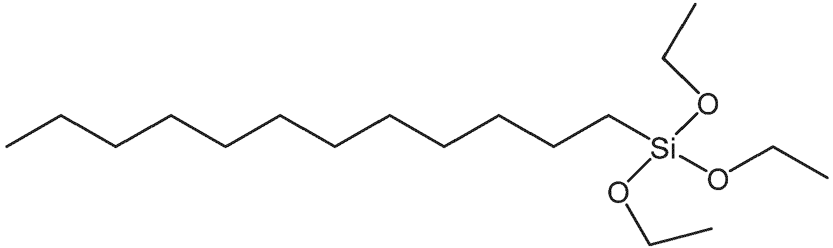
sowie Propyltrimethoxysilan, Propyltriethoxysilan, Octadecyltrimethoxysilan und/oder Octadecyltriethoxysilan.

Bei den zuvor beschriebenen organischen Siliciumverbindungen handelt es sich um reaktive Verbindungen.

In diesem Zusammenhang hat es sich als ganz besonders bevorzugt herausgestellt, wenn das Mittel als organische Siliciumverbindung (3-Aminopropyl)triethoxysilan, d.h. ein Aminopropyltriethoxysilan (AMEO), beinhaltet.

Gemäß bevorzugter Ausführungsformen der vorliegenden Erfindung ist die organische Siliciumverbindung der Formel (I), insbesondere das (3-Aminopropyl)triethoxysilan, in einer Menge von 0,01 bis 10 Gew.-%, bevorzugt von 0,02 bis 8 Gew.-%, bevorzugter von 0,05 bis 6 Gew.-%, am meisten bevorzugt von 0,1 bis 4 Gew.-%, bezogen auf das Gesamtgewicht des kosmetischen Mittels, in dem kosmetischen Mittel enthalten.

Es hat sich herausgestellt, dass auf dem keratinischen Material auch dann besonders stabile und gleichmäßige Filme erhalten werden konnten, wenn das Mittel zwei strukturell voneinander verschiedene organische Siliciumverbindungen, die jeweils ein bis drei Siliciumatome enthalten, enthält.

Das Mittel ist dadurch gekennzeichnet, dass es mindestens eine organische Silicumverbindungen der Formel (I) und mindestens eine organische Silicumverbindung der Formel (IV) enthält.

In einer explizit ganz besonders bevorzugten Ausführungsform ist ein Mittel dadurch gekennzeichnet, dass es mindestens eine organische Silicumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Silicumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein Mittel dadurch gekennzeichnet, dass das Mittelbezogen auf das Gesamtgewicht des Mittels - enthält:
- 0,5 bis 5 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem Mittel enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem Mittel enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Im Rahmen der vorliegenden Erfindung sind Angaben in Gew.-% - falls nicht anders angegeben - immer bezogen auf das Gesamtgewicht des kosmetischen Mittels.

Gemäß der vorliegenden Erfindung ist das kosmetische Mittel ein Fluid. Unter einem Fluid soll im vorliegenden Fall eine nicht-gasförmige, fließfähige Masse verstanden werden. In dem Fluid ist das mehrwertige Metallkation, insbesondere zweiwertige Metallkation, in einer bestimmten Stoffmengenkonzentration enthalten.

Als zweiten erfindungswesentlichen Bestandteil enthält das kosmetische Mittel zur Behandlung eines keratinischen Materials ein mehrwertiges Metallkation, umfassend Lanthan in Form von Lanthanchlorid. Im Zuge der zu dieser Erfindung führenden Arbeiten hat sich herausgestellt, dass es zur Lösung der der Erfindung zugrunde liegenden Aufgabe insbesondere von Vorteil ist, wenn die organische Siliciumverbindungen, beispielsweise 3-(Triethoxysilyl)-N-[3-(triethoxysilyl)propyl]-1-propanamin, d.h. ein Bis(triethoxysilylpropyl)amin, mit einem zweiwertigen Metallkation, umfassend Lanthan in Form von Lanthanchlorid, kombiniert wird.

Es wurde gefunden, dass die Kombination aus Bis(triethoxysilylpropyl)amin mit zweiwertigen Metallkation, umfassend Lanthan in Form von Lanthanchlorid, Haare vom Auswaschen künstlicher Haarfarben schützt. Ebenso wird, besonders bei oxidativ geschädigtem Haar, die Haaroberfläche hydrophober und die Denaturierungstemperatur, die Haarquellung sowie Zug-Dehnungsparameter verbessert bzw. vorteilhaft verändert. Durch die Verbesserung dieser Parameter wird das geschädigte Haar in seinen physikalischen Eigenschaften wieder an das Niveau von ungeschädigtem Haar angenähert und zeigt somit an, dass die Haarstruktur restabilisiert wurde.

Gemäß einer bevorzugten Ausführungsform beträgt der pH-Wert des kosmetischen Mittels 1 bis 9, bevorzugt 1,5 bis 8, bevorzugter 2 bis 7, noch bevorzugter 2,5 bis 6 und am meisten bevorzugt 3 bis 5.

Gemäß einer bevorzugten Ausführungsform der vorliegenden Erfindung enthält das kosmetische Mittel mehrere Tenside als Komponente c), bevorzugter sind zwei strukturell voneinander verschiedene Tenside in dem kosmetischen Mittel enthalten. Es ist insbesondere bevorzugt, dass das kosmetische Mittel zwei strukturell voneinander verschiedene Tenside enthält, wobei bevorzugt das kosmetische Mittel zwei strukturell voneinander verschiedene kationische Tenside, zwei voneinander verschiedene anionische Tenside, ein kationisches Tensid und ein nichtionisches Tensid, oder ein anionisches Tensid und ein nichtionisches Tensid enthält.

Das Mittel zur Behandlung eines keratinischen Materials kann insbesondere ein Mittel zur Reinigung eines keratinischen Materials, ein Mittel zur Pflege eines keratinischen Materials, ein Mittel zur Pflege und Reinigung eines keratinischen Materials und/oder umfassen. Das kosmetische Mittel ist ein Farbschutzschutzshampoo oder ein Farbschutz-Conditioner.

Gemäß weiterer bevorzugter Ausführungsformen der vorliegenden Erfindung enthält das kosmetische Mittel als Komponente c) mindestens ein kationisches Tensid. Dieses ist besonders bevorzugt ein kationisches Tensid der Formel (V), 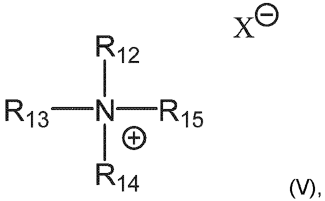 worin
- R₁₂, R₁₃, R₁₄: unabhängig voneinander für eine C1-C6-Alkylgruppe, eine C2-C6-Alkenylgruppe oder eine C2-C6-Hydroxyalkylgruppe stehen,
- R₁₅: für eine C8-C28-Alkylgruppe, bevorzugt eine C10-C22-Alkylgruppe steht und
- X-: für ein physiologisch verträgliches Anion steht,
und/oder das kosmetische Mittel enthält bevorzugt mindestens ein kationisches Tensid der Formel (VI), 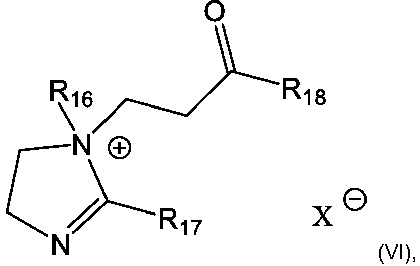 worin
- R₁₆: für eine C1-C6-Alkylgruppe steht
- R₁₇, R₁₈: unabhängig voneinander für eine C7-C27-Alkylgruppe, bevorzugt eine C10-C22-Alkylgruppe stehen und
- X-: für ein physiologisch verträgliches Anion steht,
und/oder das kosmetische Mittel enthält bevorzugt mindestens ein kationisches Tensid der Formel (VII), 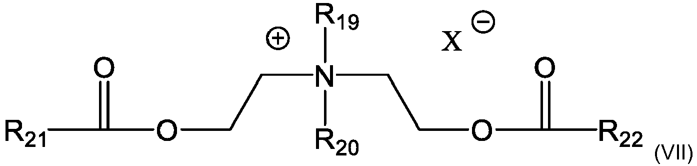 worin
- R₁₉, R₂₀: unabhängig voneinander für eine C1-C6-Alkylgruppe oder eine C2-C6-Hydroxyalkylgruppe stehen,
- R₂₁, R₂₂: unabhängig voneinander für eine C7-C27-Alkylgruppe, bevorzugt eine C10-C22-Alkylgruppe stehen und
- X-: für ein physiologisch verträgliches Anion steht,
und/oder das kosmetische Mittel enthält bevorzugt mindestens ein kationisches Tensid der Formel (VIII),

NR₂₃R₂₄R₂₅ (VIII)

worin
- R₂₃, R₂₄: unabhängig voneinander für eine C1-C6-Alkylgruppe, eine C2-C6-Alkenylgruppe oder eine C2-C6-Hydroxyalkylgruppe stehen, und
- R₂₅: für eine C8-C28-Alkylgruppe, bevorzugt eine C10-C22-Alkylgruppe steht.

Bei den kationischen Tensiden der Formel (VIII) handelt es sich um Aminderivate, sogenannte Pseudoquats. Die organischen Reste R₂₃, R₂₄ und R₂₅ sind dabei unmittelbar an das Stickstoffatom gebunden. Im sauren pH-Bereich werden diese kationisiert, d.h. das Stickstoffatom wird dann protoniert. Als Gegenionen bieten sich die physiologisch verträglichen Gegenionen an. Als besonders bevorzugt bietet sich bei den kationischen Tensiden der Formel (VIII) Steamidopropyl Dimethylamine an.

Gemäß einer bevorzugten Ausführungsform der vorliegenden Erfindung beträgt die Menge an kationischem Tensid 0,1 bis 30 Gew.-%, bevorzugt 0,5 bis 20 Gew.-%, bevorzugter 1 bis 10 Gew.-%, bezogen auf das Gesamtgewicht des kosmetischen Mittels.

Gemäß einer bevorzugten Ausführungsform der vorliegenden Erfindung umfasst das kationische Tensid eine hydrophobe Kopfgruppe mit einer kationischen Ladung und einen oder zwei hydrophobe Endteile, wobei das hydrophobe Endteil oder die hydrophoben Endteile geradkettige oder verzweigte, gesättigte oder ein- oder mehrfach ungesättigte Alkylgruppen darstellen, die bevorzugt eine Kettenlänge von C6 bis C30, bevorzugter C8 bis C26, besonders bevorzugt C10 bis C22, aufweisen. Gemäß einer weiteren bevorzugten Ausführungsform weist das kationische Tensid eine Esterfunktion, eine Etherfunktion, eine Ketonfunktion, eine Alkoholfunktion oder eine Amidfunktion auf.

Gemäß einer bevorzugten Ausführungsform der vorliegenden Erfindung enthält das kosmetische Mittel als eine weitere Komponente c) ein nichtionisches Tensid. Dieses umfasst bevorzugt ein nichtionisches Tensid ausgewählt aus der Gruppe bestehend aus
- Alkylglucamid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylfructosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylglucosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, und
- Alkylalkoholalkoxylat der Formel R₁₀(OR₁₁)ₘOH, in der R₁₀ eine lineare oder verzweigte C₆ bis C₂₂, bevorzugt C₁₀ bis C₁₈, bevorzugter C₁₂ bis C₁₆ Alkylgruppe, R₁₁ eine C₂ bis C₄, bevorzugt eine C₂ Alkylgruppe, und m 1 bis 10, bevorzugt 2 bis 6, bevorzugter 2 bis 6, darstellen.

Gemäß bevorzugter Ausführungsformen der vorliegenden Erfindung sind ein oder mehrere anionische Tenside als eine Komponente c) in dem kosmetischen Mittel enthalten, die bevorzugt ausgewählt ist aus der Gruppe bestehend aus
- geradkettigen oder verzweigten, gesättigten oder ein- oder mehrfach ungesättigten Alkylsulfonaten mit 8 bis 24, bevorzugt 12 bis 22, bevorzugter 16 bis 18 C-Atomen,
- linearen Alpha-Olefinsulfonaten mit 8 bis 24, bevorzugt 12 bis 22, bevorzugter 16 bis 18 C-Atomen,
- Alkylsulfaten und Alkylpolyglykolethersulfaten der Formel R₉-O-(CH₂-CH₂O)ₙ-SO₃X, in der R₉ bevorzugt für einen geradkettigen oder verzweigten, gesättigten oder ein- oder mehrfach ungesättigten Alkyl- oder Alkenlyrest mit 8 bis 24, bevorzugt 12 bis 22, bevorzugter 16 bis 18 Kohlenstoffatomen, n für 0 oder 1 bis 12, bevorzugter 2 bis 4 und X für ein Alkali- oder Erdalkalimetallion oder für protoniertes Triethanolamin oder das Ammonium-Ion steht,
- geradkettigen oder verzweigten, gesättigten oder ein- oder mehrfach ungesättigten Alkylcarbonsäuren mit 8 bis 24, bevorzugt 12 bis 22, bevorzugter 16 bis 18 C-Atomen,
- geradkettigen oder verzweigten, gesättigten oder ein- oder mehrfach ungesättigten Alkylphosphaten mit 8 bis 24, bevorzugt 12 bis 22, bevorzugter 16 bis 18 C-Atomen,
- Alkylisethionat, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe, insbesondere Natriumcocoylisethionat,
- Alkylglycosidcarbonsäuren, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsulfosuccinaten, dessen zwei Alkylgruppen ausgewählt sind aus gleichen oder verschiedenen, verzweigte oder unverzweigten C₂ bis C₁₂, bevorzugt C₄ bis C₁₀, bevorzugter C₆ bis C₈ Alkylgruppen,
- Alkyltauraten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsarcosinaten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Sulfonate ungesättigter Fettsäuren mit 8 bis 24, bevorzugt 12 bis 22, bevorzugter 16 bis 18 C-Atomen und 1 bis 6 Doppelbindungen,
wobei das Gegenion des anionischen Tensids ein Alkali- oder Erdalkalimetallion oder ein protoniertes Triethanolamin oder das Ammonium-Ion ist.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten.

Ganz besonders bevorzugt enthält die Tensidmischung aus anionischen und
amphoteren/zwitterionischen Tensiden Natriumlaurylethersulfat (INCI: Sodium Laureth Sulfate) und ganz besonders bevorzugt Natriumlaurylethersulfat mit 2 Ethylenoxideinheiten.

Amphotere Tenside, welche auch als zwitterionische Tenside bezeichnet werden, werden solche oberflächenaktiven Verbindungen genannt, die im Molekül mindestens eine quartäre Ammoniumgruppe und mindestens eine -COO⁻ - oder -SO₃⁻ -Gruppe tragen. Unter amphoteren/zwitterionischen Tensiden werden auch solche oberflächenaktiven Verbindungen verstanden, die außer einer C₈ - C₂₄ -Alkyl- oder-Acylgruppe mindestens eine freie Aminogruppe und mindestens eine -COOH- oder -SOsH-Gruppe enthalten und zur Ausbildung innerer Salze befähigt sind.

Gemäß einer bevorzugten Ausführungsform der vorliegenden Erfindung enthält das kosmetische Mittel als eine weitere Komponente c) mindestens ein amphoteres Tensid. Bevorzugt sind die amphoteren Tenside in dem kosmetischen Mittel ausgewählt aus der Gruppe bestehend aus
- Alkylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylamphodiacetat oder Alkylamphodiacetat, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, mit einem Alkali- oder Erdalkalimetallgegenion, und
- Alkylamidopropylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe.

Zu den insbesondere geeigneten amphoteren/zwitterionischen Tensiden zählen die unter der INCI-Bezeichnung bekannten Tenside Cocamidopropylbetain und Disodium Cocoamphodiacetate.

Gemäß einer bevorzugten Ausführungsform der vorliegenden Erfindung ist das nichtionische Tensid ausgewählt aus der Gruppe bestehend aus
- Alkylglucamid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylfructosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylglucosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylalkoholalkoxylat der Formel R₁₀(OR₁₁)ₘOH, in der R₁₀ eine lineare oder verzweigte C₆ bis C₂₂, bevorzugt C₁₀ bis C₁₈, bevorzugter C₁₂ bis C₁₆ Alkylgruppe, R₁₁ eine C₂ bis C₄, bevorzugt eine C₂ Alkylgruppe, und m 1 bis 10, bevorzugt 2 bis 6, bevorzugter 2 bis 6, darstellen, und
- Alkylester der Formel R₁₂COOR₁₃, in der R₁₂ eine lineare oder verzweigte C₆ bis C₂₂, bevorzugt C₁₀ bis C₁₈, bevorzugter C₁₂ bis C₁₆ Alkylgruppe, R₁₃ eine C₁ bis C₄, bevorzugt eine C₂ Alkylgruppe, darstellen.

Im Folgenden werden weitere Bestandteile der Haarbehandlungsmittel beschrieben, die neben den zuvor beschriebenen zwingenden Inhaltstoffen in den Mitteln enthalten sein können.

Es kann bevorzugt sein, dass das Mittel zur Behandlung eines keratinischen Materials ferner 0,001 bis 20 Gew.-% mindestens einer quaternären Verbindung umfasst. Dies gilt insbesondere für Mittel zur Pflege eines keratinischen Materials und für Mittel zur Pflege und Reinigung eines keratinischen Materials.

Es ist bevorzugt, dass die mindestens eine quaternäre Verbindung ausgewählt aus mindestens einer der Gruppen bestehend aus
i) der Monoalkylquats und/oder
ii) der Esterquats und/oder
iii) der quaternären Imidazoline der Formel (Tkat2), 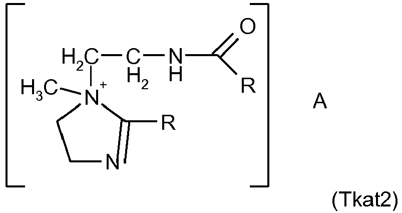 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iv) der Amidoamine und/oder kationisierten Amidoamine und/oder
v) Poly(methacryloyloxyethyltrimethylammoniumverbindungen) und/oder;
vi) quaternisierten Cellulose-Derivaten, insbesondere Polyquaternium 10, Polyquaternium-24, Polyquaternium-27, Polyquaternium-67, Polyquaternium-72, und/oder
vii) kationischen Alkylpolyglycosiden und/oder
viii) kationisiertem Honig und/oder
ix) kationischen Guar-Derivaten und/oder
x) Chitosan und/oder
xi) polymeren Dimethyldiallylammoniumsalzen und deren Copolymeren mit Estern und Amiden von Acrylsäure und Methacrylsäure, insbesondere Polyquaternium-7 und/oder
xii) Copolymeren des Vinylpyrrolidons mit quaternierten Derivaten des Dialkylaminoalkylacrylats und -methacrylats, insbesondere Polyquaternium-11 und/oder
xiii) Vinylpyrrolidon-Vinylimidazoliummethochlorid-Copolymeren, insbesondere Polyquaternium-16 und/oder
xiv) quaterniertem Polyvinylalkohol und/oder
xv) Polyquaternium-74,
sowie Mischungen hiervon.

Es ist insbesondere bevorzugt, dass das Haarbehandlungsmittel ein kationisches Hompolymer, welches unter die INCI-Bezeichnung Polyquaternium-37 fällt, als quaternäre Verbindungen enthält.

Es kann bevorzugt sein, dass das Mittel zur Behandlung eines keratinischen Materials ferner eine festigende Verbindung, vorzugsweise ausgewählt aus der Gruppe bestehend aus Wachsen, synthetischen Polymeren und Mischungen daraus, umfasst.

Die synthetischen Polymere lassen sich in kationische, anionische, nichtionische und amphotere festigende Polymere unterteilen.

Geeignete synthetische Polymere umfassen beispielsweise Polymere mit den folgenden INCI-Bezeichnungen: Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/Hydroxyesters Acrylates Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/ltaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer. Ebenso geeinet sind Celluloseether, wie Hydroxypropylcellulose, Hydroxyethylcellulose und Methylhydroxypropylcellulose.

Auch Homopolyacrylsäure (INCI: Carbomer), die im Handel unter dem Namen Carbopol^{®} in unterschiedlichen Ausführungen erhältlich ist, ist als festigende Verbindung geeignet.

Bevorzugt umfasst die festigende Verbindung ein Vinylpyrrolidon-haltiges Polymer. Besonders bevorzugt umfasst die festigende Verbindung ein Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI) und Mischungen daraus.

Eine ebenfalls bevorzugte festigende Verbindung ist Octylacrylamide/Acrylates/Butylaminoethyl Methacrylate Copolymer (INCI), welches unter der Bezeichnung "Amphomer^{®}" von Akzo Nobel vertrieben wird.

Entsprechend ist es besonders bevorzugt, dass die festigende Verbindung ein synthetisches Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI), Octylacrylamide/Acrylates/Butylaminoethyl Methacrylate Copolymer (INCI) und Mischungen daraus umfasst.

Die kosmetische Zusammensetzung kann zusätzlich oder alternativ zu einem synthetischen Polymer mindestens ein natürliches oder synthetisches Wachs, welches einen Schmelzpunkt von über 37 °C aufweist, als festigende Verbindung enthalten.

Als natürliche oder synthetische Wachse können feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie zum Beispiel Bienenwachse und andere Insektenwachse, Walrat, Schellackwachs, Wollwachs und Bürzelfett, weiterhin Mineralwachse, wie zum Beispiel Ceresin und Ozokerit oder die petrochemischen Wachse, wie zum Beispiel Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, zum Beispiel Montanesterwachse, Sasolwachse und hydrierte Jojobawachse, einsetzbar.

Weiterhin sind zusätzlich zu den zwingend enthaltenen Komponenten die Triglyceride gesättigter und gegebenenfalls hydroxylierter C16-30-Fettsäuren, wie zum Beispiel gehärtete Triglyceridfette (hydriertes Palmöl, hydriertes Kokosöl, hydriertes Rizinusöl), Glyceryltribehenat oder Glyceryltri-12-hydroxystearat in den kosmetischen Mitteln geeignet.

Die Wachskomponenten können auch aus der Gruppe der Ester aus gesättigten, unverzweigten Alkancarbonsäuren einer Kettenlänge von 22 bis 44 C-Atomen und gesättigten, unverzweigten Alkoholen einer Kettenlänge von 22 bis 44 C-Atomen ausgewählt werden, sofern die Wachskomponente oder die Gesamtheit der Wachskomponenten bei Raumtemperatur fest sind. Auch Silikonwachse, zum Beispiel Stearyltrimethylsilan/Stearylalkohol sind gegebenenfalls vorteilhaft.

Natürliche, chemisch modifizierte und synthetische Wachse können alleine oder in Kombination eingesetzt werden. Es können somit auch mehrere Wachse eingesetzt werden. Weiterhin ist auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax^{®} GP 200 (eine Mischung von Stearylalkohol und Polyethylenglykolstearat mit einem Schmelzpunkt von 47-51 °C; Hersteller: Croda) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für einsetzbare Mischungen.

Bevorzugt ist das Wachs ausgewählt aus Carnaubawachs (INCI: Copernicia Cerifera Cera) Bienenwachs (INCI: Beeswax), Petrolatum (INCI), mikrokristallinem Wachs und insbesondere Gemischen daraus.

Bevorzugte Mischungen umfassen die Kombination von Carnaubawachs (INCI: Copernicia Cerifera Cera), Petrolatum und mikrokristallinem Wachs oder die Kombination von Bienenwachs (INCI: Beeswax) und Petrolatum.

Das Wachs oder die Wachskomponenten sollten bei 25 °C fest sein und sollen im Bereich von > 37 °C schmelzen.

Das Mittel zur Behandlung eines keratinischen Materials enthält die festigende Verbindung vorzugsweise in einer Gesamtmenge von 0,5 bis 50 Gew.-%, bevorzugt 1 bis 40 Gew.-%, weiter bevorzugt 1,5 bis 30 31

Gew.-%, noch mehr bevorzugt 2 bis 25 Gew.-%, bezogen auf das Gesamtgewicht der kosmetischen Zusammensetzung.

Weitere geeignete Inhaltsstoffe umfassen Proteinhydrolysate, Aminosäuren, Oligopetide, Vitamine, Provitamine, Vitaminvorstufen, Betaine, Biochinone, Purin(derivate), Pflegestoffe, Pflanzenextrakte, Silikone, Esteröle, UV-Lichtschutzfilter, Strukturierungsmittel, Verdickungsmittel, pH-Stellmittel, Quellmittel, Antischuppenwirkstoffe, Komplexbildner, Trübungsmittel, Perlglanzmittel, Pigmente, Stabilisierungsmittel, Treibmittel, Antioxidantien, Parfümöle und/oder Konservierungsmittel.

In den bevorzugten Ausführungsformen 1 bis 14 werden die organischen Siliciumverbindungen der folgenden Tabelle mit folgenden Komponenten der folgenden Tabelle in einem erfindungsgemäßen kosmetischen Mittel miteinander kombiniert.

| | Silanverbindung | weiterer Inhaltsstoff |
|---|---|---|
| 27 | Dodecyltrimethoxysilan | 0,01 bis 1 mol/L zweiwertiges Magnesium |
| 28 | Dodecyltriethoxysilan | 0,01 bis 1 mol/L zweiwertiges Magnesium |
| 29 | (3-Aminopropyl)trimethoxysilan | 0,01 bis 1 mol/L zweiwertiges Calcium |
| 30 | (3-Aminopropyl)triethoxysilan | 0,01 bis 1 mol/L zweiwertiges Calcium |
| 31 | (2-Aminoethyl)trimethoxysilan | 0,01 bis 1 mol/L zweiwertiges Calcium |
| 32 | (2-Aminoethyl)triethoxysilan | 0,01 bis 1 mol/L zweiwertiges Calcium |
| 33 | (3-Dimethylaminopropyl)trimethoxysilan | 0,01 bis 1 mol/L zweiwertiges Calcium |
| 34 | (3-Dimethylaminopropyl)triethoxysilan | 0,01 bis 1 mol/L zweiwertiges Calcium |
| 35 | (2-Dimethylaminoethyl)trimethoxysilan | 0,01 bis 1 mol/L zweiwertiges Calcium |
| 36 | (2-Dimethylaminoethyl)triethoxysilan | 0,01 bis 1 mol/L zweiwertiges Calcium |
| 37 | 3-(Trimethoxysilyl)-N-[3-(trimethoxysilyl)propyl]-1-propanamin | 0,01 bis 1 mol/L zweiwertiges Calcium |
| 38 | 3-(Triethoxysilyl)-N-[3-(triethoxysilyl)propyl]-1-propanamin | 0,01 bis 1 mol/L zweiwertiges Calcium |
| 39 | N-Methyl-3-(trimethoxysilyl)-N-[3-(trimethoxysilyl)propyl]-1-propanamin | 0,01 bis 1 mol/L zweiwertiges Calcium |
| 40 | N-Methyl-3-(triethoxysilyl)-N-[3-(triethoxysilyl)propyl]-1-propanamin | 0,01 bis 1 mol/L zweiwertiges Calcium |
| 41 | 2-[Bis[3-(trimethoxysilyl)propyl]amino]-ethanol | 0,01 bis 1 mol/L zweiwertiges Calcium |
| 42 | 2-[Bis[3-(triethoxysilyl)propyl]amino]-ethanol | 0,01 bis 1 mol/L zweiwertiges Calcium |
| 43 | 3-(Trimethoxysilyl)-N,N-bis[3-(trimethoxysilyl)propyl]-1-propanamin | 0,01 bis 1 mol/L zweiwertiges Calcium |
| 44 | 3-(Triethoxysilyl)-N,N-bis[3-(triethoxysilyl)propyl]-1-propanamin | 0,01 bis 1 mol/L zweiwertiges Calcium |
| 45 | N1,N1-Bis[3-(trimethoxysilyl)propyl]-1,2-ethandiamin | 0,01 bis 1 mol/L zweiwertiges Calcium |
| 46 | N1,N1-Bis[3-(triethoxysilyl)propyl]-1,2-ethandiamin | 0,01 bis 1 mol/L zweiwertiges Calcium |
| 47 | N,N-Bis[3-(trimethoxysilyl)propyl]-2-propen-1-amin | 0,01 bis 1 mol/L zweiwertiges Calcium |
| 48 | N,N-Bis[3-(triethoxysilyl)propyl]-2-propen-1-amin | 0,01 bis 1 mol/L zweiwertiges Calcium |
| 49 | Methyltrimethoxysilan | 0,01 bis 1 mol/L zweiwertiges Calcium |
| 50 | Methyltriethoxysilan | 0,01 bis 1 mol/L zweiwertiges Calcium |
| 51 | Ethyltrimethoxysilan | 0,01 bis 1 mol/L zweiwertiges Calcium |
| 52 | Ethyltriethoxysilan | 0,01 bis 1 mol/L zweiwertiges Calcium |
| 53 | Octyltrimethoxysilan | 0,01 bis 1 mol/L zweiwertiges Calcium |
| 54 | Octyltriethoxysilan | 0,01 bis 1 mol/L zweiwertiges Calcium |
| 55 | Dodecyltrimethoxysilan | 0,01 bis 1 mol/L zweiwertiges Calcium |
| 56 | Dodecyltriethoxysilan | 0,01 bis 1 mol/L zweiwertiges Calcium |
| 57 | (3-Aminopropyl)trimethoxysilan | 0,01 bis 1 mol/L zweiwertiges Zink |
| 58 | (3-Aminopropyl)triethoxysilan | 0,01 bis 1 mol/L zweiwertiges Zink |
| 59 | (2-Aminoethyl)trimethoxysilan | 0,01 bis 1 mol/L zweiwertiges Zink |
| 60 | (2-Aminoethyl)triethoxysilan | 0,01 bis 1 mol/L zweiwertiges Zink |
| 61 | (3-Dimethylaminopropyl)trimethoxysilan | 0,01 bis 1 mol/L zweiwertiges Zink |
| 62 | (3-Dimethylaminopropyl)triethoxysilan | 0,01 bis 1 mol/L zweiwertiges Zink |
| 63 | (2-Dimethylaminoethyl)trimethoxysilan | 0,01 bis 1 mol/L zweiwertiges Zink |
| 64 | (2-Dimethylaminoethyl)triethoxysilan | 0,01 bis 1 mol/L zweiwertiges Zink |
| 65 | 3-(Trimethoxysilyl)-N-[3-(trimethoxysilyl)propyl]-1-propanamin | 0,01 bis 1 mol/L zweiwertiges Zink |
| 66 | 3-(Triethoxysilyl)-N-[3-(triethoxysilyl)propyl]-1-propanamin | 0,01 bis 1 mol/L zweiwertiges Zink |
| 67 | N-Methyl-3-(trimethoxysilyl)-N-[3-(trimethoxysilyl)propyl]-1-propanamin | 0,01 bis 1 mol/L zweiwertiges Zink |
| 68 | N-Methyl-3-(triethoxysilyl)-N-[3-(triethoxysilyl)propyl]-1-propanamin | 0,01 bis 1 mol/L zweiwertiges Zink |
| 69 | 2-[Bis[3-(trimethoxysilyl)propyl]amino]-ethanol | 0,01 bis 1 mol/L zweiwertiges Zink |
| 70 | 2-[Bis[3-(triethoxysilyl)propyl]amino]-ethanol | 0,01 bis 1 mol/L zweiwertiges Zink |
| 71 | 3-(Trimethoxysilyl)-N,N-bis[3-(trimethoxysilyl)propyl]-1-propanamin | 0,01 bis 1 mol/L zweiwertiges Zink |
| 72 | 3-(Triethoxysilyl)-N,N-bis[3-(triethoxysilyl)propyl]-1-propanamin | 0,01 bis 1 mol/L zweiwertiges Zink |
| 73 | N1,N1-Bis[3-(trimethoxysilyl)propyl]-1 ,2-ethandiamin | 0,01 bis 1 mol/L zweiwertiges Zink |
| 74 | N1,N1-Bis[3-(triethoxysilyl)propyl]-1,2-ethandiamin | 0,01 bis 1 mol/L zweiwertiges Zink |
| 75 | N,N-Bis[3-(trimethoxysilyl)propyl]-2-propen-1-amin | 0,01 bis 1 mol/L zweiwertiges Zink |
| 76 | N,N-Bis[3-(triethoxysilyl)propyl]-2-propen-1-amin | 0,01 bis 1 mol/L zweiwertiges Zink |
| 77 | Methyltrimethoxysilan | 0,01 bis 1 mol/L zweiwertiges Zink |
| 78 | Methyltriethoxysilan | 0,01 bis 1 mol/L zweiwertiges Zink |
| 79 | Ethyltrimethoxysilan | 0,01 bis 1 mol/L zweiwertiges Zink |
| 80 | Ethyltriethoxysilan | 0,01 bis 1 mol/L zweiwertiges Zink |
| 81 | Octyltrimethoxysilan | 0,01 bis 1 mol/L zweiwertiges Zink |
| 82 | Octyltriethoxysilan | 0,01 bis 1 mol/L zweiwertiges Zink |
| 83 | Dodecyltrimethoxysilan | 0,01 bis 1 mol/L zweiwertiges Zink |
| 84 | Dodecyltriethoxysilan | 0,01 bis 1 mol/L zweiwertiges Zink |
| 85 | (3-Aminopropyl)trimethoxysilan | 0,01 bis 1 mol/L zweiwertiges Zirkonium |
| 86 | (3-Aminopropyl)triethoxysilan | 0,01 bis 1 mol/L zweiwertiges Zirkonium |
| 87 | (2-Aminoethyl)trimethoxysilan | 0,01 bis 1 mol/L zweiwertiges Zirkonium |
| 88 | (2-Aminoethyl)triethoxysilan | 0,01 bis 1 mol/L zweiwertiges Zirkonium |
| 89 | (3-Dimethylaminopropyl)trimethoxysilan | 0,01 bis 1 mol/L zweiwertiges Zirkonium |
| 90 | (3-Dimethylaminopropyl)triethoxysilan | 0,01 bis 1 mol/L zweiwertiges Zirkonium |
| 91 | (2-Dimethylaminoethyl)trimethoxysilan | 0,01 bis 1 mol/L zweiwertiges Zirkonium |
| 92 | (2-Dimethylaminoethyl)triethoxysilan | 0,01 bis 1 mol/L zweiwertiges Zirkonium |
| 93 | 3-(Trimethoxysilyl)-N-[3-(trimethoxysilyl)propyl]-1-propanamin | 0,01 bis 1 mol/L zweiwertiges Zirkonium |
| 94 | 3-(Triethoxysilyl)-N-[3-(triethoxysilyl)propyl]-1-propanamin | 0,01 bis 1 mol/L zweiwertiges Zirkonium |
| 95 | N-Methyl-3-(trimethoxysilyl)-N-[3-(trimethoxysilyl)propyl]-1-propanamin | 0,01 bis 1 mol/L zweiwertiges Zirkonium |
| 96 | N-Methyl-3-(triethoxysilyl)-N-[3-(triethoxysilyl)propyl]-1-propanamin | 0,01 bis 1 mol/L zweiwertiges Zirkonium |
| 97 | 2-[Bis[3-(trimethoxysilyl)propyl]amino]-ethanol | 0,01 bis 1 mol/L zweiwertiges Zirkonium |
| 98 | 2-[Bis[3-(triethoxysilyl)propyl]amino]-ethanol | 0,01 bis 1 mol/L zweiwertiges Zirkonium |
| 99 | 3-(Trimethoxysilyl)-N,N-bis[3-(trimethoxysilyl)propyl]-1-propanamin | 0,01 bis 1 mol/L zweiwertiges Zirkonium |
| 100 | 3-(Triethoxysilyl)-N,N-bis[3-(triethoxysilyl)propyl]-1-propanamin | 0,01 bis 1 mol/L zweiwertiges Zirkonium |
| 101 | N1 ,N1-Bis[3-(trimethoxysilyl)propyl]-1 ,2-ethandiamin | 0,01 bis 1 mol/L zweiwertiges Zirkonium |
| 102 | N1,N1-Bis[3-(triethoxysilyl)propyl]-1,2-ethandiamin | 0,01 bis 1 mol/L zweiwertiges Zirkonium |
| 103 | N,N-Bis[3-(trimethoxysilyl)propyl]-2-propen-1-amin | 0,01 bis 1 mol/L zweiwertiges Zirkonium |
| 104 | N,N-Bis[3-(triethoxysilyl)propyl]-2-propen-1-amin | 0,01 bis 1 mol/L zweiwertiges Zirkonium |
| 105 | Methyltrimethoxysilan | 0,01 bis 1 mol/L zweiwertiges Zirkonium |
| 106 | Methyltriethoxysilan | 0,01 bis 1 mol/L zweiwertiges Zirkonium |
| 107 | Ethyltrimethoxysilan | 0,01 bis 1 mol/L zweiwertiges Zirkonium |
| 108 | Ethyltriethoxysilan | 0,01 bis 1 mol/L zweiwertiges Zirkonium |
| 109 | Octyltrimethoxysilan | 0,01 bis 1 mol/L zweiwertiges Zirkonium |
| 110 | Octyltriethoxysilan | 0,01 bis 1 mol/L zweiwertiges Zirkonium |
| 111 | Dodecyltrimethoxysilan | 0,01 bis 1 mol/L zweiwertiges Zirkonium |
| 112 | Dodecyltriethoxysilan | 0,01 bis 1 mol/L zweiwertiges Zirkonium |
| 113 | (3-Aminopropyl)trimethoxysilan | 0,01 bis 1 mol/L dreiwertiges Lanthan |
| 114 | (3-Aminopropyl)triethoxysilan | 0,01 bis 1 mol/L dreiwertiges Lanthan |
| 115 | (2-Aminoethyl)trimethoxysilan | 0,01 bis 1 mol/L dreiwertiges Lanthan |
| 116 | (2-Aminoethyl)triethoxysilan | 0,01 bis 1 mol/L dreiwertiges Lanthan |
| 117 | (3-Dimethylaminopropyl)trimethoxysilan | 0,01 bis 1 mol/L dreiwertiges Lanthan |
| 118 | (3-Dimethylaminopropyl)triethoxysilan | 0,01 bis 1 mol/L dreiwertiges Lanthan |
| 119 | (2-Dimethylaminoethyl)trimethoxysilan | 0,01 bis 1 mol/L dreiwertiges Lanthan |
| 120 | (2-Dimethylaminoethyl)triethoxysilan | 0,01 bis 1 mol/L dreiwertiges Lanthan |
| 121 | 3-(Trimethoxysilyl)-N-[3-(trimethoxysilyl)propyl]-1-propanamin | 0,01 bis 1 mol/L dreiwertiges Lanthan |
| 122 | 3-(Triethoxysilyl)-N-[3-(triethoxysilyl)propyl]-1-propanamin | 0,01 bis 1 mol/L dreiwertiges Lanthan |
| 123 | N-Methyl-3-(trimethoxysilyl)-N-[3-(trimethoxysilyl)propyl]-1-propanamin | 0,01 bis 1 mol/L dreiwertiges Lanthan |
| 124 | N-Methyl-3-(triethoxysilyl)-N-[3-(triethoxysilyl)propyl]-1-propanamin | 0,01 bis 1 mol/L dreiwertiges Lanthan |
| 125 | 2-[Bis[3-(trimethoxysilyl)propyl]amino]-ethanol | 0,01 bis 1 mol/L dreiwertiges Lanthan |
| 126 | 2-[Bis[3-(triethoxysilyl)propyl]amino]-ethanol | 0,01 bis 1 mol/L dreiwertiges Lanthan |
| 127 | 3-(Trimethoxysilyl)-N,N-bis[3-(trimethoxysilyl)propyl]-1-propanamin | 0,01 bis 1 mol/L dreiwertiges Lanthan |
| 128 | 3-(Triethoxysilyl)-N,N-bis[3-(triethoxysilyl)propyl]-1-propanamin | 0,01 bis 1 mol/L dreiwertiges Lanthan |
| 129 | N1,N1-Bis[3-(trimethoxysilyl)propyl]-1,2-ethandiamin | 0,01 bis 1 mol/L dreiwertiges Lanthan |
| 130 | N1,N1-Bis[3-(triethoxysilyl)propyl]-1,2-ethandiamin | 0,01 bis 1 mol/L dreiwertiges Lanthan |
| 131 | N,N-Bis[3-(trimethoxysilyl)propyl]-2-propen-1-amin | 0,01 bis 1 mol/L dreiwertiges Lanthan |
| 132 | N,N-Bis[3-(triethoxysilyl)propyl]-2-propen-1-amin | 0,01 bis 1 mol/L dreiwertiges Lanthan |
| 133 | Methyltrimethoxysilan | 0,01 bis 1 mol/L dreiwertiges Lanthan |
| 134 | Methyltriethoxysilan | 0,01 bis 1 mol/L dreiwertiges Lanthan |
| 135 | Ethyltrimethoxysilan | 0,01 bis 1 mol/L dreiwertiges Lanthan |
| 136 | Ethyltriethoxysilan | 0,01 bis 1 mol/L dreiwertiges Lanthan |
| 137 | Octyltrimethoxysilan | 0,01 bis 1 mol/L dreiwertiges Lanthan |
| 138 | Octyltriethoxysilan | 0,01 bis 1 mol/L dreiwertiges Lanthan |
| 139 | Dodecyltrimethoxysilan | 0,01 bis 1 mol/L dreiwertiges Lanthan |
| 140 | Dodecyltriethoxysilan | 0,01 bis 1 mol/L dreiwertiges Lanthan |
| 141 | (3-Aminopropyl)trimethoxysilan | 0,01 bis 1 mol/L zweiwertiges Cer |
| 142 | (3-Aminopropyl)triethoxysilan | 0,01 bis 1 mol/L zweiwertiges Cer |
| 143 | (2-Aminoethyl)trimethoxysilan | 0,01 bis 1 mol/L zweiwertiges Cer |
| 144 | (2-Aminoethyl)triethoxysilan | 0,01 bis 1 mol/L zweiwertiges Cer |
| 145 | (3-Dimethylaminopropyl)trimethoxysilan | 0,01 bis 1 mol/L zweiwertiges Cer |
| 146 | (3-Dimethylaminopropyl)triethoxysilan | 0,01 bis 1 mol/L zweiwertiges Cer |
| 147 | (2-Dimethylaminoethyl)trimethoxysilan | 0,01 bis 1 mol/L zweiwertiges Cer |
| 148 | (2-Dimethylaminoethyl)triethoxysilan | 0,01 bis 1 mol/L zweiwertiges Cer |
| 149 | 3-(Trimethoxysilyl)-N-[3-(trimethoxysilyl)propyl]-1-propanamin | 0,01 bis 1 mol/L zweiwertiges Cer |
| 150 | 3-(Triethoxysilyl)-N-[3-(triethoxysilyl)propyl]-1-propanamin | 0,01 bis 1 mol/L zweiwertiges Cer |
| 151 | N-Methyl-3-(trimethoxysilyl)-N-[3-(trimethoxysilyl)propyl]-1-propanamin | 0,01 bis 1 mol/L zweiwertiges Cer |
| 152 | N-Methyl-3-(triethoxysilyl)-N-[3-(triethoxysilyl)propyl]-1-propanamin | 0,01 bis 1 mol/L zweiwertiges Cer |
| 153 | 2-[Bis[3-(trimethoxysilyl)propyl]amino]-ethanol | 0,01 bis 1 mol/L zweiwertiges Cer |
| 154 | 2-[Bis[3-(triethoxysilyl)propyl]amino]-ethanol | 0,01 bis 1 mol/L zweiwertiges Cer |
| 155 | 3-(Trimethoxysilyl)-N,N-bis[3-(trimethoxysilyl)propyl]-1-propanamin | 0,01 bis 1 mol/L zweiwertiges Cer |
| 156 | 3-(Triethoxysilyl)-N,N-bis[3-(triethoxysilyl)propyl]-1-propanamin | 0,01 bis 1 mol/L zweiwertiges Cer |
| 157 | N1,N1-Bis[3-(trimethoxysilyl)propyl]-1,2-ethandiamin | 0,01 bis 1 mol/L zweiwertiges Cer |
| 158 | N1,N1-Bis[3-(triethoxysilyl)propyl]-1,2-ethandiamin | 0,01 bis 1 mol/L zweiwertiges Cer |
| 159 | N,N-Bis[3-(trimethoxysilyl)propyl]-2-propen-1-amin | 0,01 bis 1 mol/L zweiwertiges Cer |
| 160 | N,N-Bis[3-(triethoxysilyl)propyl]-2-propen-1-amin | 0,01 bis 1 mol/L zweiwertiges Cer |
| 161 | Methyltrimethoxysilan | 0,01 bis 1 mol/L zweiwertiges Cer |
| 162 | Methyltriethoxysilan | 0,01 bis 1 mol/L zweiwertiges Cer |
| 163 | Ethyltrimethoxysilan | 0,01 bis 1 mol/L zweiwertiges Cer |
| 164 | Ethyltriethoxysilan | 0,01 bis 1 mol/L zweiwertiges Cer |
| 165 | Octyltrimethoxysilan | 0,01 bis 1 mol/L zweiwertiges Cer |
| 166 | Octyltriethoxysilan | 0,01 bis 1 mol/L zweiwertiges Cer |
| 167 | Dodecyltrimethoxysilan | 0,01 bis 1 mol/L zweiwertiges Cer |
| 168 | Dodecyltriethoxysilan | 0,01 bis 1 mol/L zweiwertiges Cer |
| 169 | Propyltrimethoxysilan | 0,01 bis 1 mol/L zweiwertiges Magnesium |
| 170 | Propyltriethoxysilan | 0,01 bis 1 mol/L zweiwertiges Magnesium |
| 171 | Hexyltrimethoxysilan | 0,01 bis 1 mol/L zweiwertiges Magnesium |
| 172 | Hexyltriethoxysilan | 0,01 bis 1 mol/L zweiwertiges Magnesium |
| 173 | Octadecyltriethoxysilan | 0,01 bis 1 mol/L zweiwertiges Magnesium |
| 174 | Octadecyltriethoxysilan | 0,01 bis 1 mol/L zweiwertiges Magnesium |
| 175 | (3-Aminopropyl)triethoxysilan + Methyltrimethoxysilan | 0,01 bis 1 mol/L zweiwertiges Magnesium |
| 176 | (3-Aminopropyl)triethoxysilan + Methyltriethoxysilan | 0,01 bis 1 mol/L zweiwertiges Magnesium |
| 177 | (3-Aminopropyl)triethoxysilan + Ethyltrimethoxysilan | 0,01 bis 1 mol/L zweiwertiges Magnesium |
| 178 | (3-Aminopropyl)triethoxysilan + Ethyltriethoxysilan | 0,01 bis 1 mol/L zweiwertiges Magnesium |
| 179 | (3-Aminopropyl)triethoxysilan + Propyltrimethoxysilan | 0,01 bis 1 mol/L zweiwertiges Magnesium |
| 180 | (3-Aminopropyl)triethoxysilan + Propyltriethoxysilan | 0,01 bis 1 mol/L zweiwertiges Magnesium |
| 181 | (3-Aminopropyl)triethoxysilan + Hexyltrimethoxysilan | 0,01 bis 1 mol/L zweiwertiges Magnesium |
| 182 | (3-Aminopropyl)triethoxysilan + Hexyltriethoxysilan | 0,01 bis 1 mol/L zweiwertiges Magnesium |
| 183 | (3-Aminopropyl)triethoxysilan + Octyltrimethoxysilan | 0,01 bis 1 mol/L zweiwertiges Magnesium |
| 184 | (3-Aminopropyl)triethoxysilan + Octyltriethoxysilan | 0,01 bis 1 mol/L zweiwertiges Magnesium |
| 185 | (3-Aminopropyl)triethoxysilan + Dodecyltrimethoxysilan | 0,01 bis 1 mol/L zweiwertiges Magnesium |
| 186 | (3-Aminopropyl)triethoxysilan + Dodecyltriethoxysilan | 0,01 bis 1 mol/L zweiwertiges Magnesium |
| 187 | (3-Aminopropyl)triethoxysilan + Octadecyltrimethoxysilan | 0,01 bis 1 mol/L zweiwertiges Magnesium |
| 188 | (3-Aminopropyl)triethoxysilan + Octadecyltriethoxysilan | 0,01 bis 1 mol/L zweiwertiges Magnesium |
| 189 | Propyltrimethoxysilan | 0,01 bis 1 mol/L zweiwertiges Calcium |
| 190 | Propyltriethoxysilan | 0,01 bis 1 mol/L zweiwertiges Calcium |
| 191 | Hexyltrimethoxysilan | 0,01 bis 1 mol/L zweiwertiges Calcium |
| 192 | Hexyltriethoxysilan | 0,01 bis 1 mol/L zweiwertiges Calcium |
| 193 | Octadecyltriethoxysilan | 0,01 bis 1 mol/L zweiwertiges Calcium |
| 194 | Octadecyltriethoxysilan | 0,01 bis 1 mol/L zweiwertiges Calcium |
| 195 | (3-Aminopropyl)triethoxysilan + Methyltrimethoxysilan | 0,01 bis 1 mol/L zweiwertiges Calcium |
| 196 | (3-Aminopropyl)triethoxysilan + Methyltriethoxysilan | 0,01 bis 1 mol/L zweiwertiges Calcium |
| 197 | (3-Aminopropyl)triethoxysilan + Ethyltrimethoxysilan | 0,01 bis 1 mol/L zweiwertiges Calcium |
| 198 | (3-Aminopropyl)triethoxysilan + Ethyltriethoxysilan | 0,01 bis 1 mol/L zweiwertiges Calcium |
| 199 | (3-Aminopropyl)triethoxysilan + Propyltrimethoxysilan | 0,01 bis 1 mol/L zweiwertiges Calcium |
| 200 | (3-Aminopropyl)triethoxysilan + Propyltriethoxysilan | 0,01 bis 1 mol/L zweiwertiges Calcium |
| 201 | (3-Aminopropyl)triethoxysilan + Hexyltrimethoxysilan | 0,01 bis 1 mol/L zweiwertiges Calcium |
| 202 | (3-Aminopropyl)triethoxysilan + Hexyltriethoxysilan | 0,01 bis 1 mol/L zweiwertiges Calcium |
| 203 | (3-Aminopropyl)triethoxysilan + Octyltrimethoxysilan | 0,01 bis 1 mol/L zweiwertiges Calcium |
| 204 | (3-Aminopropyl)triethoxysilan + Octyltriethoxysilan | 0,01 bis 1 mol/L zweiwertiges Calcium |
| 205 | (3-Aminopropyl)triethoxysilan + Dodecyltrimethoxysilan | 0,01 bis 1 mol/L zweiwertiges Calcium |
| 206 | (3-Aminopropyl)triethoxysilan + Dodecyltriethoxysilan | 0,01 bis 1 mol/L zweiwertiges Calcium |
| 207 | (3-Aminopropyl)triethoxysilan + Octadecyltrimethoxysilan | 0,01 bis 1 mol/L zweiwertiges Calcium |
| 208 | (3-Aminopropyl)triethoxysilan + Octadecyltriethoxysilan | 0,01 bis 1 mol/L zweiwertiges Calcium |
| 209 | Propyltrimethoxysilan | 0,01 bis 1 mol/L zweiwertiges Zink |
| 210 | Propyltriethoxysilan | 0,01 bis 1 mol/L zweiwertiges Zink |
| 211 | Hexyltrimethoxysilan | 0,01 bis 1 mol/L zweiwertiges Zink |
| 212 | Hexyltriethoxysilan | 0,01 bis 1 mol/L zweiwertiges Zink |
| 213 | Octadecyltriethoxysilan | 0,01 bis 1 mol/L zweiwertiges Zink |
| 214 | Octadecyltriethoxysilan | 0,01 bis 1 mol/L zweiwertiges Zink |
| 215 | (3-Aminopropyl)triethoxysilan + Methyltrimethoxysilan | 0,01 bis 1 mol/L zweiwertiges Zink |
| 216 | (3-Aminopropyl)triethoxysilan + Methyltriethoxysilan | 0,01 bis 1 mol/L zweiwertiges Zink |
| 217 | (3-Aminopropyl)triethoxysilan + Ethyltrimethoxysilan | 0,01 bis 1 mol/L zweiwertiges Zink |
| 218 | (3-Aminopropyl)triethoxysilan + Ethyltriethoxysilan | 0,01 bis 1 mol/L zweiwertiges Zink |
| 219 | (3-Aminopropyl)triethoxysilan + Propyltrimethoxysilan | 0,01 bis 1 mol/L zweiwertiges Zink |
| 220 | (3-Aminopropyl)triethoxysilan + Propyltriethoxysilan | 0,01 bis 1 mol/L zweiwertiges Zink |
| 221 | (3-Aminopropyl)triethoxysilan + Hexyltrimethoxysilan | 0,01 bis 1 mol/L zweiwertiges Zink |
| 222 | (3-Aminopropyl)triethoxysilan + Hexyltriethoxysilan | 0,01 bis 1 mol/L zweiwertiges Zink |
| 223 | (3-Aminopropyl)triethoxysilan + Octyltrimethoxysilan | 0,01 bis 1 mol/L zweiwertiges Zink |
| 224 | (3-Aminopropyl)triethoxysilan + Octyltriethoxysilan | 0,01 bis 1 mol/L zweiwertiges Zink |
| 225 | (3-Aminopropyl)triethoxysilan + Dodecyltrimethoxysilan | 0,01 bis 1 mol/L zweiwertiges Zink |
| 226 | (3-Aminopropyl)triethoxysilan + Dodecyltriethoxysilan | 0,01 bis 1 mol/L zweiwertiges Zink |
| 227 | (3-Aminopropyl)triethoxysilan + Octadecyltrimethoxysilan | 0,01 bis 1 mol/L zweiwertiges Zink |
| 228 | (3-Aminopropyl)triethoxysilan + Octadecyltriethoxysilan | 0,01 bis 1 mol/L zweiwertiges Zink |
| 229 | Propyltrimethoxysilan | 0,01 bis 1 mol/L zweiwertiges Zirkonium |
| 230 | Propyltriethoxysilan | 0,01 bis 1 mol/L zweiwertiges Zirkonium |
| 231 | Hexyltrimethoxysilan | 0,01 bis 1 mol/L zweiwertiges Zirkonium |
| 232 | Hexyltriethoxysilan | 0,01 bis 1 mol/L zweiwertiges Zirkonium |
| 233 | Octadecyltriethoxysilan | 0,01 bis 1 mol/L zweiwertiges Zirkonium |
| 234 | Octadecyltriethoxysilan | 0,01 bis 1 mol/L zweiwertiges Zirkonium |
| 235 | (3-Aminopropyl)triethoxysilan + Methyltrimethoxysilan | 0,01 bis 1 mol/L zweiwertiges Zirkonium |
| 236 | (3-Aminopropyl)triethoxysilan + Methyltriethoxysilan | 0,01 bis 1 mol/L zweiwertiges Zirkonium |
| 237 | (3-Aminopropyl)triethoxysilan + Ethyltrimethoxysilan | 0,01 bis 1 mol/L zweiwertiges Zirkonium |
| 238 | (3-Aminopropyl)triethoxysilan + Ethyltriethoxysilan | 0,01 bis 1 mol/L zweiwertiges Zirkonium |
| 239 | (3-Aminopropyl)triethoxysilan + Propyltrimethoxysilan | 0,01 bis 1 mol/L zweiwertiges Zirkonium |
| 240 | (3-Aminopropyl)triethoxysilan + Propyltriethoxysilan | 0,01 bis 1 mol/L zweiwertiges Zirkonium |
| 241 | (3-Aminopropyl)triethoxysilan + Hexyltrimethoxysilan | 0,01 bis 1 mol/L zweiwertiges Zirkonium |
| 242 | (3-Aminopropyl)triethoxysilan + Hexyltriethoxysilan | 0,01 bis 1 mol/L zweiwertiges Zirkonium |
| 243 | (3-Aminopropyl)triethoxysilan + Octyltrimethoxysilan | 0,01 bis 1 mol/L zweiwertiges Zirkonium |
| 244 | (3-Aminopropyl)triethoxysilan + Octyltriethoxysilan | 0,01 bis 1 mol/L zweiwertiges Zirkonium |
| 245 | (3-Aminopropyl)triethoxysilan + Dodecyltrimethoxysilan | 0,01 bis 1 mol/L zweiwertiges Zirkonium |
| 246 | (3-Aminopropyl)triethoxysilan + Dodecyltriethoxysilan | 0,01 bis 1 mol/L zweiwertiges Zirkonium |
| 247 | (3-Aminopropyl)triethoxysilan + Octadecyltrimethoxysilan | 0,01 bis 1 mol/L zweiwertiges Zirkonium |
| 248 | (3-Aminopropyl)triethoxysilan + Octadecyltriethoxysilan | 0,01 bis 1 mol/L zweiwertiges Zirkonium |
| 249 | Propyltrimethoxysilan | 0,01 bis 1 mol/L dreiwertiges Lanthan |
| 250 | Propyltriethoxysilan | 0,01 bis 1 mol/L dreiwertiges Lanthan |
| 251 | Hexyltrimethoxysilan | 0,01 bis 1 mol/L dreiwertiges Lanthan |
| 252 | Hexyltriethoxysilan | 0,01 bis 1 mol/L dreiwertiges Lanthan |
| 253 | Octadecyltriethoxysilan | 0,01 bis 1 mol/L dreiwertiges Lanthan |
| 254 | Octadecyltriethoxysilan | 0,01 bis 1 mol/L dreiwertiges Lanthan |
| 1 | (3-Aminopropyl)triethoxysilan + Methyltrimethoxysilan | 0,01 bis 1 mol/L dreiwertiges Lanthan |
| 2 | (3-Aminopropyl)triethoxysilan + Methyltriethoxysilan | 0,01 bis 1 mol/L dreiwertiges Lanthan |
| 3 | (3-Aminopropyl)triethoxysilan + Ethyltrimethoxysilan | 0,01 bis 1 mol/L dreiwertiges Lanthan |
| 4 | (3-Aminopropyl)triethoxysilan + Ethyltriethoxysilan | 0,01 bis 1 mol/L dreiwertiges Lanthan |
| 5 | (3-Aminopropyl)triethoxysilan + Propyltrimethoxysilan | 0,01 bis 1 mol/L dreiwertiges Lanthan |
| 6 | (3-Aminopropyl)triethoxysilan + Propyltriethoxysilan | 0,01 bis 1 mol/L dreiwertiges Lanthan |
| 7 | (3-Aminopropyl)triethoxysilan + Hexyltrimethoxysilan | 0,01 bis 1 mol/L dreiwertiges Lanthan |
| 8 | (3-Aminopropyl)triethoxysilan + Hexyltriethoxysilan | 0,01 bis 1 mol/L dreiwertiges Lanthan |
| 9 | (3-Aminopropyl)triethoxysilan + Octyltrimethoxysilan | 0,01 bis 1 mol/L dreiwertiges Lanthan |
| 10 | (3-Aminopropyl)triethoxysilan + Octyltriethoxysilan | 0,01 bis 1 mol/L dreiwertiges Lanthan |
| 11 | (3-Aminopropyl)triethoxysilan + Dodecyltrimethoxysilan | 0,01 bis 1 mol/L dreiwertiges Lanthan |
| 12 | (3-Aminopropyl)triethoxysilan + Dodecyltriethoxysilan | 0,01 bis 1 mol/L dreiwertiges Lanthan |
| 13 | (3-Aminopropyl)triethoxysilan + Octadecyltrimethoxysilan | 0,01 bis 1 mol/L dreiwertiges Lanthan |
| 14 | (3-Aminopropyl)triethoxysilan + Octadecyltriethoxysilan | 0,01 bis 1 mol/L dreiwertiges Lanthan |

Die Kombinationen der obigen Tabelle stellen Wirkstoffkombinationen dar, die in kosmetischen Mitteln mit weiteren, oben beschriebenen Komponenten kombiniert werden.

Die Wirkstoffkombination aus den organischen Siliciumverbindung und dem mehrwertigen Metallkation kann bereits im Mittel zur Behandlung eines keratinischen Materials enthalten sein. In dieser Ausführungsform wird das Mittel zur Behandlung eines keratinischen Materials bereits in anwendungsbereiter Form vertrieben. Um eine möglichst lagerstabile Formulierung bereitstellen zu können, wird das Mittel selbst bevorzugt wasserarm oder wasserfrei konfektioniert.

Alternativ wird die mindestens eine organische Siliciumverbindung maximal 12 Stunden, bevorzugt maximal 6 Stunden, mehr bevorzugt maximal 3 Stunde, noch mehr bevorzugt maximal 1 Stunde vor Anwendung des Mittels zur Behandlung eines keratinischen Materials einer Basis umfassend alle Inhaltsstoffe des Mittels zur Behandlung eines keratinischen Materials mit Ausnahme der mindestens einen organischen Siliciumverbindung zugefügt.

Ferner werden alternativ die organische Siliciumverbindung und die weitere Komponente b) einem kosmetischen Produkt erst kurz vor der Anwendung, d.h. 1 Minute bis 12 Stunden, bevorzugt 2 Minuten bis 6 Stunden, besonders bevorzugt 1 Minute bis 3 Stunden, insbesondere bevorzugt 1 Minute bis 1 Stunde, zugegeben.

In einer weiteren Alternative werden die organischen Siliciumverbindungen einer wässrigen Lösung zugegeben, welche auf das Haar appliziert wird und im zweiten Schritt wird eine wässrige Lösung oder ein kosmetisches Mittel, welches die weitere Komponente b) enthält, auf das Haar aufgetragen und das Haar somit nachbehandelt.

Beispielsweise kann der Anwender ein Mittel (α), welches die organische Siliciumverbindung(en) enthält, zunächst mit einem Mittel (β), welches die restlichen Inhaltsstoffe des Mittels zur Behandlung eines keratinischen Materials umfasst, verrühren oder verschütteln. Diese Mischung aus (α) und (β) kann der Anwender nun - entweder direkt nach ihrer Herstellung oder nach einer kurzen Reaktionszeit von 1 Minute bis 20 Minuten - auf die keratinischen Materialien applizieren. Das Mittel (β) kann Wasser enthalten, insbesondere Wasser in einer Menge > 30 Gew.-%, bezogen auf das Gesamtgewicht des Mittels zur Behandlung keratinischer Materialien.

Bezüglich weiterer bevorzugter Ausführungsformen der Verwendung gilt mutatis mutandis das zu den kosmetischen Mitteln Gesagte.

## Patentansprüche

1. Kosmetisches Mittel zur Behandlung eines keratinischen Materials, wobei das kosmetische Mittel ein Fluid darstellt und
a) mindestens eine organische Siliciumverbindung, wobei die mindestens eine organische Siliciumverbindung eine Verbindung der Formel (I) und mindestens eine organische Siliciumverbindung der Formel (IV) enthält,
wobei in der organischen Siliciumverbindung der Formel (I)
R₁R₂N-L-Si(OR₃)ₐ(R₄)_{b} (I),
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht, und
wobei in der organischen Siliciumverbindung der Formel (IV)
R₉Si(OR₁₀)ₖ(R₁₁)ₘ (IV),
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht, und
b) mindestens ein mehrwertiges Metallkation umfassend Lanthan in Form von Lanthanchlorid, in einer Stoffmengenkonzentration von 0,001 mol/L bis 2 mol/L, bevorzugt 0,005 mol/L bis 1,5 mol/L, bevorzugter 0,01 bis 1 mol/L, am meisten bevorzugt 0,02 bis 0,5 mol/L, umfasst.

2. Kosmetisches Mittel zur Behandlung eines keratinischen Materials gemäß Anspruch 1, **dadurch gekennzeichnet,**
**dass** die mindestens eine organische Siliciumverbindung ferner eine Verbindung der Formel (II) enthält,
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} und R_{6"} unabhängig voneinander für eine C₁-C₆-Alkylgruppe stehen,
- A, A', A", A‴ und Aʺʺ unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III) stehen
-
(Aʺʺ)-Si(R₆")_{d'}(OR₅")_{c"} (III),
- c für eine ganze Zahl von 1 bis 3 steht,
- d für die ganze Zahl 3 - c steht,
- c' für eine ganze Zahl von 1 bis 3 steht,
- d` für die ganze Zahl 3 - c` steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Kosmetisches Mittel zur Behandlung eines keratinischen Materials gemäß einem der Ansprüche 1 oder 2,
**dadurch gekennzeichnet,**
**dass** das Mittel zur Behandlung eines keratinischen Materials mindestens eine organische Siliciumverbindung der Formel (I) enthält, die ausgewählt ist aus der Gruppe bestehend aus
- (3-Aminopropyl)trimethoxysilan
- (3-Aminopropyl)triethoxysilan
- (2-Aminoethyl)trimethoxysilan und
- (2-Aminoethyl)triethoxysilan

4. Kosmetisches Mittel zur Behandlung eines keratinischen Materials gemäß einem der Ansprüche 1 bis 3,
**dadurch gekennzeichnet,**
**dass** das Mittel zur Behandlung eines keratinischen Materials mindestens eine organische Siliciumverbindung der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1 ,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und
- N,N-Bis[3-(triethoxysilyl)propyl]-2-Propen-1-amin.

5. Kosmetisches Mittel zur Behandlung eines keratinischen Materials gemäß einem der Ansprüche 1 bis 4, **dadurch gekennzeichnet, dass** die organische Siliciumverbindung in einer Menge von 0,01 bis 10 Gew.-%, bevorzugt von 0,02 bis 9 Gew.-%, bevorzugter von 0,05 bis 8 Gew.-%, noch bevorzugter von 0,1 bis 7 Gew.-%, bezogen auf das Gesamtgewicht des kosmetischen Mittels, in dem kosmetischen Mittel enthalten ist,
und oder **dadurch gekennzeichnet, dass** die organische Siliciumverbindung der Formel (I) (3-Aminopropyl)triethoxysilan ist.

6. Kosmetisches Mittel zur Behandlung eines keratinischen Materials gemäß einem der Ansprüche 1 bis 5, **dadurch gekennzeichnet, dass** das Mittel zur Behandlung eines keratinischen Materials ferner mindestens eine organische Siliciumverbindung der Formel (IV) enthält,
R₉Si(OR₁₀)ₖ(R₁₁)ₘ (IV),
die ausgewählt ist aus der Gruppe bestehend aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan
- Dodecyltriethoxysilan
- Octadecyltrimethoxysilan und
- Octadecyltriethoxysilan.

7. Kosmetisches Mittel zur Behandlung eines keratinischen Materials gemäß einem der Ansprüche 1 bis 6, **dadurch gekennzeichnet, dass** der pH-Wert des kosmetischen Mittels 1 bis 9, bevorzugt 1,5 bis 8, bevorzugter 2 bis 7, noch bevorzugter 2,5 bis 6 und am meisten bevorzugt 3 bis 5 beträgt.

8. Kosmetisches Mittel zur Behandlung eines keratinischen Materials gemäß einem der Ansprüche 1 bis 7, **dadurch gekennzeichnet, dass** als eine Komponente c) mindestens ein Tensid in dem kosmetischen Mittel enthalten ist, wobei bevorzugt das Tensid ein anionisches, kationische, nichtionisches oder amphoteres Tensid ist, bevorzugter enthält das kosmetische Mittel zwei strukturell voneinander verschiedene Tenside, wobei noch bevorzugter das kosmetische Mittel zwei strukturell voneinander verschiedene kationische Tenside, zwei voneinander verschiedene anionische Tenside, ein kationisches Tensid und ein nichtionisches Tensid, oder ein anionisches Tensid und ein nichtionisches Tensid enthält.

9. Kosmetisches Mittel zur Behandlung eines keratinischen Materials gemäß einem der Ansprüche 1 bis 8, **dadurch gekennzeichnet, dass** das Mittel zur Behandlung eines keratinischen Materials- bezogen auf das Gesamtgewicht des Mittels zur Behandlung eines keratinischen Materials - enthält:
- 0,5 bis 3 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan und (2-Aminoethyl)triethoxysilan, und
- 3,2 bis 7 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

## Claims

1. A cosmetic agent for treating a keratinous material, wherein the cosmetic agent is a fluid and comprises
a) at least one organosilicon compound, wherein the at least one organosilicon compound contains a compound of formula (I) and at least one organosilicon compound of formula (IV),
where, in the organosilicon compound of formula (I),
R₁R₂N-L-Si(OR₃)ₐ(R₄)_{b} (I),
- R₁ and R₂ both represent a hydrogen atom,
- L represents a linear, divalent C₁-C₆ alkylene group, preferably a propylene group (-CH₂-CH₂-CH₂-) or an ethylene group (-CH₂-CH₂-),
- R₃ and R₄ independently represent a methyl group or an ethyl group,
- a represents the number 3, and
- b represents the number 0, and
where, in the organosilicon compound of formula (IV),
R₉Si(OR₁₀)ₖ(R₁₁)ₘ (IV),
- R₉ represents a C₁-C₁₂ alkyl group,
- R₁₀ represents a hydrogen atom or a C₁-C₆ alkyl group,
- R₁₁ represents a C₁-C₆ alkyl group, and
- k represents an integer from 1 to 3, and
- m represents the integer 3 - k, and
b) at least one multivalent metal cation comprising lanthanum in the form of lanthanum chloride, in a molar concentration of 0.001 mol/L to 2 mol/L, preferably 0.005 mol/L to 1.5 mol/L, more preferably 0.01 to 1 mol/L, most preferably 0.02 to 0.5 mol/L.

2. The cosmetic agent for treating a keratinous material according to claim 1,
**characterized in that**
the at least one organosilicon compound further contains a compound of formula (II),
where, in the organosilicon compound of formula (II),
(R₅O)_{c}(R₆)_{d}Si-(A)ₑ-[NR₇-(A')_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} and R_{6"} independently represent a C₁-C₆ alkyl group,
- A, A', A", A‴ and Aʺʺ independently represent a linear or branched, divalent C₁-C₂₀ alkylene group,
- R₇ and R₈ independently represent a hydrogen atom, a C₁-C₆-alkyl group, a hydroxy C₁-C₆-alkyl group, a C₂-C₆-alkenyl group, an amino C₁-C₆-alkyl group, or a grouping of formula (III),
(Aʺʺ)-Si(R₆")_{d"}(OR₅")_{c"} (III),
- c represents an integer from 1 to 3,
- d represents the integer 3 - c,
- c' represents an integer from 1 to 3,
- d' represents the integer 3 - c',
- c" represents an integer from 1 to 3,
- d" represents the integer 3 - c",
- e represents 0 or 1,
- f represents 0 or 1,
- g represents 0 or 1,
- h represents 0 or 1,
with the proviso that at least one of the functional groups e, f, g and h is different from 0.

3. The cosmetic agent for treating a keratinous material according to one of claims 1 or 2,
**characterized in that**
the agent for treating a keratinous material contains at least one organosilicon compound of formula (I), which is selected from the group consisting of
- (3-aminopropyl)trimethoxysilane,
- (3-aminopropyl)triethoxysilane,
- (2-aminoethyl)trimethoxysilane, and
- (2-aminoethyl)triethoxysilane

4. The cosmetic agent for treating a keratinous material according to one of claims 1 to 3,
**characterized in that**
the agent for treating a keratinous material contains at least one organosilicon compound of formula (II), which is selected from the group consisting of
- 3-(trimethoxysilyl)-N-[3-(trimethoxysilyl)propyl]-1-propanamine
- 3-(triethoxysilyl)-N-[3-(triethoxysilyl)propyl]-1-propanamine
- N-methyl-3-(trimethoxysilyl)-N-[3-(trimethoxysilyl)propyl]-1-propanamine
- N-methyl-3-(triethoxysilyl)-N-[3-(triethoxysilyl)propyl]-1-propanamine
- 2-[bis[3-(trimethoxysilyl)propyl]amino]ethanol
- 2-[bis[3-(triethoxysilyl)propyl]amino]ethanol
- 3-(trimethoxysilyl)-N,N-bis[3-(trimethoxysilyl)propyl]-1-propanamine
- 3-(triethoxysilyl)-N,N-bis[3-(triethoxysilyl)propyl]-1-propanamine
- N1,N1-bis[3-(trimethoxysilyl)propyl]-1,2-ethanediamine,
- N1,N1-bis[3-(triethoxysilyl)propyl]-1,2-ethanediamine,
- N,N-bis[3-(trimethoxysilyl)propyl]-2-propen-1-amine, and
- N,N-bis[3-(triethoxysilyl)propyl]-2-propen-1-amine.

5. The cosmetic agent for treating a keratinous material according to one of claims 1 to 4, **characterized in that** the organosilicon compound is contained in the cosmetic agent in an amount of from 0.01 to 10 wt.%, preferably from 0.02 to 9 wt.%, more preferably from 0.05 to 8 wt.%, even more preferably from 0.1 to 7 wt.%, based on the total weight of the cosmetic agent,
and or **characterized in that** the organosilicon compound of formula (I) is (3-aminopropyl)triethoxysilane.

6. The cosmetic agent for treating a keratinous material according to one of claims 1 to 5, **characterized in that** the agent for treating a keratinous material further contains at least one organosilicon compound of formula (IV),
R₉Si(OR₁₀)ₖ(R₁₁)ₘ (IV),
which is selected from the group consisting of
- methyltrimethoxysilane
- methyltriethoxysilane
- ethyltrimethoxysilane
- ethyltriethoxysilane
- propyltrimethoxysilane
- propyltriethoxysilane
- hexyltrimethoxysilane
- hexyltriethoxysilane
- octyltrimethoxysilane
- octyltriethoxysilane
- dodecyltrimethoxysilane
- dodecyltriethoxysilane
- octadecyltrimethoxysilane and
- octadecyltriethoxysilane.

7. The cosmetic agent for treating a keratinous material according to one of claims 1 to 6, **characterized in that** the pH value of the cosmetic agent is 1 to 9, preferably 1.5 to 8, more preferably 2 to 7, even more preferably 2.5 to 6 and most preferably 3 to 5.

8. The cosmetic agent for treating a keratinous material according to one of claims 1 to 7, **characterized in that** at least one surfactant is contained in the cosmetic agent as a component c), wherein the surfactant is preferably an anionic, cationic, nonionic or amphoteric surfactant, more preferably the cosmetic agent contains two structurally different surfactants, wherein even more preferably the cosmetic agent contains two structurally different cationic surfactants, two different anionic surfactants, a cationic surfactant and a nonionic surfactant, or an anionic surfactant and a nonionic surfactant.

9. The cosmetic agent for treating a keratinous material according to one of claims 1 to 8, **characterized in that** the agent for treating a keratinous material contains, based on the total weight of the agent for treating a keratinous material:
- 0.5 to 3 wt.% of at least one first organosilicon compound selected from the group consisting of (3-aminopropyl)trimethoxysilane, (3-aminopropyl)triethoxysilane, (2-aminoethyl)trimethoxysilane and (2-aminoethyl)triethoxysilane, and
- 3.2 to 7 wt.% of at least one second organosilicon compound selected from the group consisting of methyltrimethoxysilane, methyltriethoxysilane, ethyltrimethoxysilane, ethyltriethoxysilane, propyltrimethoxysilane, propyltriethoxysilane, hexyltrimethoxysilane, octyltrimethoxysilane, octyltriethoxysilane, dodecyltrimethoxysilane, dodecyltriethoxysilane, octadecyltrimethoxysilane and octadecyltriethoxysilane.

## Revendications

1. Agent cosmétique pour le traitement d'une matière kératinique, dans lequel l'agent cosmétique est un fluide et comprend
a) au moins un composé organique de silicium, dans lequel l'au moins un composé organique de silicium contient un composé de formule (I) et au moins un composé organique de silicium de formule (IV),
où, dans le composé organique de silicium de formule (I)
R₁R₂N-L-Si(OR₃)ₐ(R₄)_{b} (I),
- R₁, R₂ représentent tous deux un atome d'hydrogène,
- L représente un groupe alkylène en C₁-C₆ bivalent linéaire, de préférence un groupe propylène (-CH₂-CH₂-CH₂-) ou un groupe éthylène (-CH₂-CH₂-),
- R₃, R₄ représentent, indépendamment l'un de l'autre, un groupe méthyle ou un groupe éthyle,
- a représente le nombre 3 et
- b représente le nombre 0, et
où, dans le composé organique de silicium de formule (IV)
R₉Si(OR₁₀)ₖ(R₁₁)ₘ (IV),
- R₉ représente un groupe alkyle en C₁-C₁₂,
- R₁₀ représente un atome d'hydrogène ou un groupe alkyle en C₁-C₆,
- R₁₁ représente un groupe alkyle en C₁-C₆
- k représente un nombre entier allant de 1 à 3, et
- m représente le nombre entier 3 - k, et
b) au moins un cation métallique polyvalent comprenant du lanthane sous forme de chlorure de lanthane, à une concentration molaire allant de 0,001 mol/l à 2 mol/l, de préférence de 0,005 mol/l à 1,5 mol/l, plus préférablement de 0,01 à 1 mol/l, de manière particulièrement préférée de 0,02 à 0,5 mol/l.

2. Agent cosmétique pour le traitement d'une matière kératinique selon la revendication 1,
**caractérisé en ce**
**que** l'au moins un composé organique de silicium contient en outre un composé de formule (II),
où, dans le composé organique de silicium de formule (II)
(R₅O)_{c}(R₆)_{d}Si-(A)ₑ-[NR₇-(A')_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} et R_{6"} représentent, indépendamment les uns des autres, un groupe alkyle en C₁-C₆,
- A, A', A", A‴ et A"" représentent, indépendamment les uns des autres, un groupe alkylène en C₁-C₂₀ bivalent, linéaire ou ramifié,
- R₇ et R₈ représentent, indépendamment l'un de l'autre, un atome d'hydrogène, un groupe alkyle en C₁-C₆, un groupe hydroxy alkyle en C₁-C₆, un groupe alcényle en C₂-C₆, un groupe amino alkyle en C₁-C₆ ou un groupement de formule (III)
-
(A"")-Si(R₆")_{d"}(OR_{5"})_{c"} (III),
- c représente un nombre entier de 1 à 3,
- d représente le nombre entier 3 - c,
- c' représente un nombre entier de 1 à 3,
- d' représente le nombre entier 3 - c`,
- c" représente un nombre entier de 1 à 3,
- d" représente le nombre entier 3 - c",
- e représente 0 ou 1,
- f représente 0 ou 1,
- g représente 0 ou 1,
- h représente 0 ou 1,
à condition qu'au moins l'un des radicaux parmi e, f, g et h soit différent de 0.

3. Agent cosmétique pour le traitement d'une matière kératinique selon l'une des revendications 1 ou 2,
**caractérisé en ce**
**que** l'agent pour le traitement d'une matière kératinique contient au moins un composé organique de silicium de formule (I) qui est choisi dans le groupe constitué de
- (3-aminopropyl)triméthoxysilane
- (3-aminopropyl)triéthoxysilane
- (2-aminoéthyl)triméthoxysilane et
- (2-aminoéthyl)triéthoxysilane.

4. Agent cosmétique pour le traitement d'une matière kératinique selon l'une des revendications 1 à 3,
**caractérisé en ce**
**que** l'agent pour le traitement d'une matière kératinique contient au moins un composé organique de silicium de formule (II) qui est choisi dans le groupe constitué de
- 3-(triméthoxysilyl)-N-[3-(triméthoxysilyl)propyl]-1-propanamine
- 3-(triéthoxysilyl)-N-[3-(triéthoxysilyl)propyl]-1-propanamine
- N-méthyl-3-(triméthoxysilyl)-N-[3-(triméthoxysilyl)propyl]-1-propanamine
- N-méthyl-3-(triéthoxysilyl)-N-[3-(triéthoxysilyl)propyl]-1-propanamine
- 2-[bis[3-(triméthoxysilyl)propyl]amino]-éthanol
- 2-[bis[3-(triéthoxysilyl)propyl]amino]-éthanol
- 3-(triméthoxysilyl)-N,N-bis[3-(triméthoxysilyl)propyl]-1-propanamine
- 3-(triéthoxysilyl)-N,N-bis[3-(triéthoxysilyl)propyl]-1-propanamine
- N1,N1-bis[3-(triméthoxysilyl)propyl]-1,2-éthanediamine,
- N1,N1-bis[3-(triéthoxysilyl)propyl]-1,2-éthanediamine,
- N,N-bis[3-(triméthoxysilyl)propyl]-2-propén-1-amine et
- N,N-bis[3-(triéthoxysilyl)propyl]-2-propén-1-amine.

5. Agent cosmétique pour le traitement d'une matière kératinique selon l'une des revendications 1 à 4, **caractérisé en ce que** le composé organique de silicium est contenu dans l'agent cosmétique en une quantité allant de 0,01 à 10 % en poids, de préférence de 0,02 à 9 % en poids, plus préférablement de 0,05 à 8 % en poids, encore plus préférablement de 0,1 à 7 % en poids, par rapport au poids total de l'agent cosmétique,
et ou **caractérisé en ce que** le composé organique de silicium de formule (I) est le (3-aminopropyl)triéthoxysilane.

6. Agent cosmétique pour le traitement d'une matière kératinique selon l'une des revendications 1 à 5, **caractérisé en ce que** l'agent pour le traitement d'une matière kératinique contient en outre au moins un composé organique de silicium de formule (IV)
R₉Si(OR₁₀)ₖ(R₁₁)ₘ (IV),
lequel est choisi dans le groupe constitué de
- méthyltriméthoxysilane
- méthyltriéthoxysilane
- éthyltriméthoxysilane
- éthyltriéthoxysilane
- propyltriméthoxysilane
- propyltriéthoxysilane
- hexyltriméthoxysilane
- hexyltriéthoxysilane
- octyltriméthoxysilane
- octyltriéthoxysilane
- dodécyltriméthoxysilane
- dodécyltriéthoxysilane
- octadécyltriméthoxysilane et
- octadécyltriéthoxysilane.

7. Agent cosmétique pour le traitement d'une matière kératinique selon l'une des revendications 1 à 6, **caractérisé en ce que** le pH de l'agent cosmétique va de 1 à 9, de préférence de 1,5 à 8, plus préférablement de 2 à 7, encore plus préférablement de 2,5 à 6, et de manière particulièrement préférée de 3 à 5.

8. Agent cosmétique pour le traitement d'une matière kératinique selon l'une des revendications 1 à 7, **caractérisé en ce qu'**au moins un tensioactif est contenu en tant que composant c) dans l'agent cosmétique, dans lequel, de préférence, le tensioactif est un tensioactif anionique, cationique, non ionique ou amphotère, plus préférablement, l'agent cosmétique contient deux tensioactifs structurellement différents l'un de l'autre, dans lequel, encore plus préférablement, l'agent cosmétique contient deux tensioactifs cationiques structurellement différents l'un de l'autre, deux tensioactifs anioniques différents l'un de l'autre, un tensioactif cationique et un tensioactif non ionique, ou un tensioactif anionique et un tensioactif non ionique.

9. Agent cosmétique pour le traitement d'une matière kératinique selon l'une des revendications 1 à 8, **caractérisé en ce que** l'agent pour le traitement d'une matière kératinique contient, par rapport au poids total de l'agent pour le traitement d'une matière kératinique :
- 0,5 à 3 % en poids d'au moins un premier composé organique de silicium qui est choisi dans le groupe constitué de (3-aminopropyl)triméthoxysilane, (3-aminopropyl)triéthoxysilane, (2-aminoéthyl)triméthoxysilane et (2-aminoéthyl)triéthoxysilane, et
- 3,2 à 7 % en poids d'au moins un second composé organique de silicium qui est choisi dans le groupe constitué de méthyltriméthoxysilane, méthyltriéthoxysilane, éthyltriméthoxysilane, éthyltriéthoxysilane, propyltriméthoxysilane, propyltriéthoxysilane, hexyltriméthoxysilane, octyltriméthoxysilane octyltriéthoxysilane, dodécyltriméthoxysilane, dodécyltriéthoxysilane, octadécyltriméthoxysilane et octadécyltriéthoxysilane.
